# EUROPEAN PATENT SPECIFICATION

(11) **EP 1 980 148 B1**
(45) Date of publication and mention of the grant of the patent: **20.03.2013**
(21) Application number: 07707732.9
(22) Date of filing: 30.01.2007
(51) Int. Cl.: A01K 67/027, C12Q 1/68, G01N 33/15, G01N 33/50, C12N 15/09

(54) **GENETICALLY MODIFIED ANIMAL AND USE THEREOF**
GENETISCH MODIFIZIERTES TIER UND SEINE VERWENDUNG
ANIMAL GENETIQUEMENT MODIFIE ET SON UTILISATION

(30) Priority: 31.01.2006 JP 2006022913
(43) Date of publication of application: 15.10.2008
(73) Proprietor: Takeda Pharmaceutical Company Limited, Osaka-shi, Osaka 541-0045 (JP)
(72) Inventor: KAISHO, Yoshihiko, c/o Takeda Pharmaceutical Company Limited, Fujisawa Kanagawa 251-0012 (JP); TAKETOMI, Shigehisa, Nishinomiya-city, Hyogo 662-0095 (JP)
(74) Representative: Weiss, Wolfgang
(86) International application number: PCT/JP2007/051514
(87) International publication number: WO 2007/088857

(56) References cited:
- EP-A1- 1 479 768
- WO-A1-02/43477
- WO-A1-03/068959
- WO-A1-2005/015990
- JP-A- 09 028 384
- STENEBERG PAR ET AL: "The FFA receptor GPR40 links hyperinsulinemia, hepatic steatosis, and impaired glucose homeostasis in mouse" April 2005 (2005-04), CELL METABOLISM, VOL. 1, NR. 4, PAGE(S) 245-258 URL , XP002596040 ISSN: 1550-4131 * the whole document *
- NAGASUMI KAE ET AL: "Overexpression of GPR40 in Pancreatic beta-Cells Augments Glucose-Stimulated Insulin Secretion and Improves Glucose Tolerance in Normal and Diabetic Mice" DIABETES, vol. 58, no. 5, May 2009 (2009-05), pages 1067-1076, XP002595946 ISSN: 0012-1797
- TOMITA T. ET AL.: 'GPR40 gene expression in human pancreas and insulinoma' BIOCHEM. BIOPHYS. RES. COMMUN. vol. 338, no. 4, 2005, pages 1788 - 1790, XP005207264

## Description

### Technical Field

The present invention relates to a GPR40 gene transgenic non-human mammal, and a screening method for an insulin secretion and/or glucose tolerance regulatory drug, an anti-obesity drug, an insulin sensitivity regulatory drug and the like using the mammals and the like. Described is also a non-human mammal deficient in the expression of the GPR40 gene.

### Background of the Invention

GPR40 was reported as an orphan G protein coupled receptor with unidentified ligand in 1997 (nonpatent document 1, patent document 1); later it was reported that this receptor is expressed in the pancreas, and that the ligand therefor is a free fatty acid (patent document 2). Furthermore, free fatty acids have been shown to promote insulin secretion from pancreatic P cell-derived cell lines via the receptor (nonpatent document 2, patent document 3).
From these findings, it is thought that compounds that act specifically on GPR40 can control plasma insulin concentrations; GPR40 agonists are expected to serve as prophylactic/therapeutic drugs having a new mechanism of action on diabetes mellitus. However, much remains unclear as to the functions of GPR40 in vivo.

Pancreatic P cell disorder underlies diabetes mellitus; improvement of impaired insulin secretion is also therapeutically effective. Therefore, to elucidate the roles of the GPR40 gene in vivo is important not only from the viewpoint of basic research, but also from the viewpoint of drug discovery research.
In functional analysis of genes, genetically modified animals such as transgenic mice and knockout mice are highly useful. GPR40 gene transgenic mice and GPR40 gene-knockout mice have also been reported (nonpatent document 3, patent documents 425) According thereto, the GPR40 knockout mice are resistant to impaired glucose tolerance due to high-fat diet load, whereas the GPR40 gene transgenic mice develop diabetes mellitus accompanied by impaired insulin secretion.
patent document 1: WO2000/22129
patent document 2: WO02/057783
patent document 3: WO03/068959
patent document 4: WO2005/015990
patent document 5: EP1479768
nonpatent document 1: Biochem. Biophys. Res. Commun., 1997, 239(2): 543-7
nonpatent document 2: Nature, 2003, 422(6928): 173-6
nonpatent document 3: Cell Metabol., 2005, 1: 245-58

### Disclosure of the Invention

### Problems to be Solved by the Invention

It is an object of the present invention to generate and analyze GPR40 gene transgenic animals and GPR40 gene knockout animals, with the aim of elucidating the functions of the GPR40 gene in vivo, and to provide a screening and drug efficacy evaluation system for prophylactic/therapeutic drugs for diabetes mellitus using the genetically modified animals thus obtained.

### Means of Solving the Problems

The GPR40 gene transgenic mice, generated by Edlund et al. and Walker et al. (nonpatent document 3, patent document 4 above), had GPR40 overexpressed under control of IPF1 promoter. In contrast, the present inventors generated transgenic (Tg) mice by introducing the human GPR40 gene under the control of an insulin promoter. As a result, the Tg mice resulted in augmented insulin secretion and improved glucose tolerance compared with the corresponding non-transgenic mice. Hence, the phenotypes of the transgenic mice according to the present invention support the results for the insulin secretion augmentation by GPR40 that have been shown in vitro, and also support the concept of preventing/treating diabetes mellitus by enhancing the activity of GPR40.
Meanwhile, the present inventors also succeeded in generating GPR40 gene knockout (KO) animals having the GPR40 gene deleted using homologous recombination. In the congenic strain obtained by back-crossing the KO mice with the C57BL/6J strain, no remarkable phenotypes were observed, compared with wild type mice.
The present inventors conducted further investigations based on these findings, and developed the present invention.

Accordingly, the present invention provides the subject matter defined in the claims. The present disclosure further provides:
[1] a transgenic mouse retaining a DNA that encodes an exogenous GPR40 in an expressible state, wherein (1) the insulin secretion capacity has been increased, and/or (2) the glucose tolerance has been improved, compared with the corresponding non-transgenic mouse, or a portion of the living body thereof,
[2] the mouse according to [1] above, wherein the DNA that encodes an exogenous GPR40 is under the control of an insulin promoter, or a portion of the living body thereof,
[3] the mouse according to [1] above, wherein the exogenous GPR40 has the same or substantially the same amino acid sequence as the amino acid sequence shown by SEQ ID NO:2, or a portion of the living body thereof,
[4] a screening method for an GPR40 agonist or a GPR40 antagonist, comprising applying a test compound to the mouse according to [1] above or a portion of the living body thereof, and determining the GPR40 agonist activity or GPR40 antagonist activity,
[5] a screening method for an (1) insulin secretion and/or (2) glucose tolerance regulatory drug, comprising applying a test compound to the mouse according to [1] above or a portion of the living body thereof, and measuring the (1) insulin secretion and/or (2) glucose tolerance,
[6] a mouse fertilized egg retaining a DNA that encodes an exogenous GPR40 under the control of an insulin promoter,
[7] the mouse according to [1] above, wherein the exogenous GPR40 is heterogeneous to the mouse, and the mouse is deficient in the expression of the endogenous GPR40 gene, or a portion of the living body thereof,
[8] the mouse according to [7] above, wherein the heterogeneous GPR40 is derived from human, or a portion of the living body thereof,
[9] a screening method for a heterogeneous GPR40 agonist or a heterogeneous GPR40 antagonist, comprising applying a test compound to the mouse according to [7] above or a portion of the living body thereof, and determining the GPR40 agonist activity or GPR40 antagonist activity,
[10] a screening method for an (1) insulin secretion and/or (2) glucose tolerance regulatory drug in a heterogeneous mammal, comprising applying a test compound to the mouse according to
[7] above or a portion of the living body thereof, and measuring the (1) insulin secretion and/or (2) glucose tolerance,
[11] a pathologic condition model mouse resulting from mating of the mouse according to [1] above and another pathologic condition model mouse, or a portion of the living body thereof,
[12] a pathologic condition model mouse resulting from drug induction or stress loading on the mouse according to [1] above, or a portion of the living body thereof,
[13] a screening method for a prophylactic/therapeutic substance for a disease accompanied by a pathologic condition, comprising applying a test compound to the mouse according to
[11] or [12] above or a portion of the living body thereof, and determining the amelioration of the pathologic condition,
[14] the method according to [13] above, wherein the pathologic condition or disease is metabolic syndrome or one or more symptoms thereof, and the like.

### [Effect of the Invention]

The GPR40 gene modified animals of the present invention have effects of exhibiting phenotypes that are more reflective of normal GPR40 functions, and can serve as screening and drug efficacy evaluation systems for GPR40 action regulatory drugs in vivo.

### [Brief Description of the Drawings]

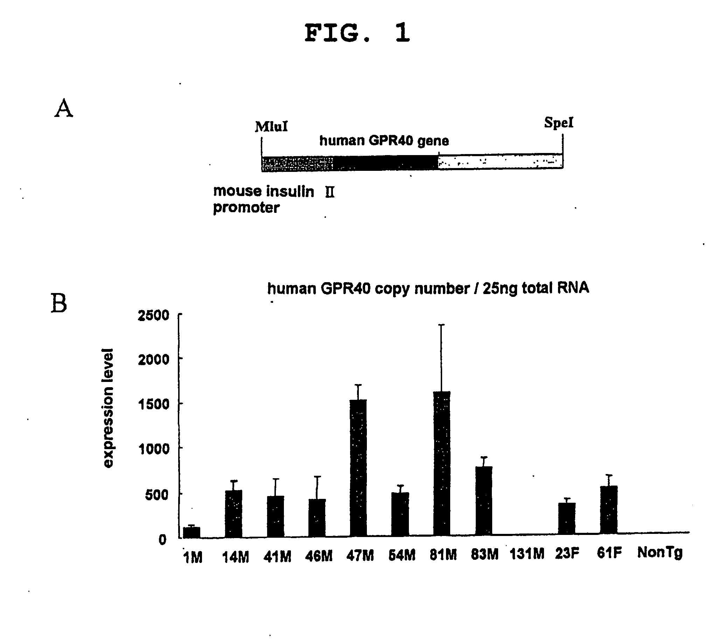

[FIG. 1] (A) A scheme showing the structure of a human GPR40 gene expression unit. This is a structure wherein the human GPR40 gene, including a poly(A)+ addition signal, is joined downstream of mouse insulin II gene promoter. (B) A graph showing the expression of the human GPR40 gene in the pancreases of the transgenic mice. Shown are the expression level of the human GPR40 gene in the pancreases of 8-week-old mice of various strains. Each bar and error bar indicate a mean and a standard deviation, respectively (Mean±SE, n=3 to 6). Note that the error bars for the 47M strain, 54M strain, 23F strain, and 61F strain indicate deviations from means (Mean±SE, n=2). NonTg: control mice.

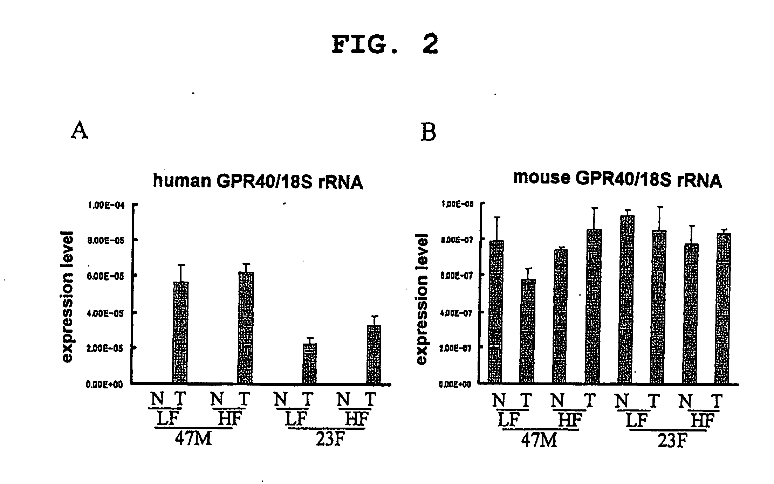

[FIG. 2] Graphs showing the expression of the human (A) and mouse (B) GPR40 genes in isolated islets of Langerhans in the transgenic mouse 47M strain and 23F strain. The animals were loaded with a low-fat diet or high-fat diet for 8 weeks from 8 week-old. The expression level of the human or mouse GPR40 gene is shown as a ratio to the 18S ribosome RNA gene expression level. Each bar and error bar indicate a mean and a standard deviation, respectively (Mean±SE, n=3 to 4). LF: low-fat diet load group, HF: high-fat diet load group, N: control mice, T: transgenic mice.

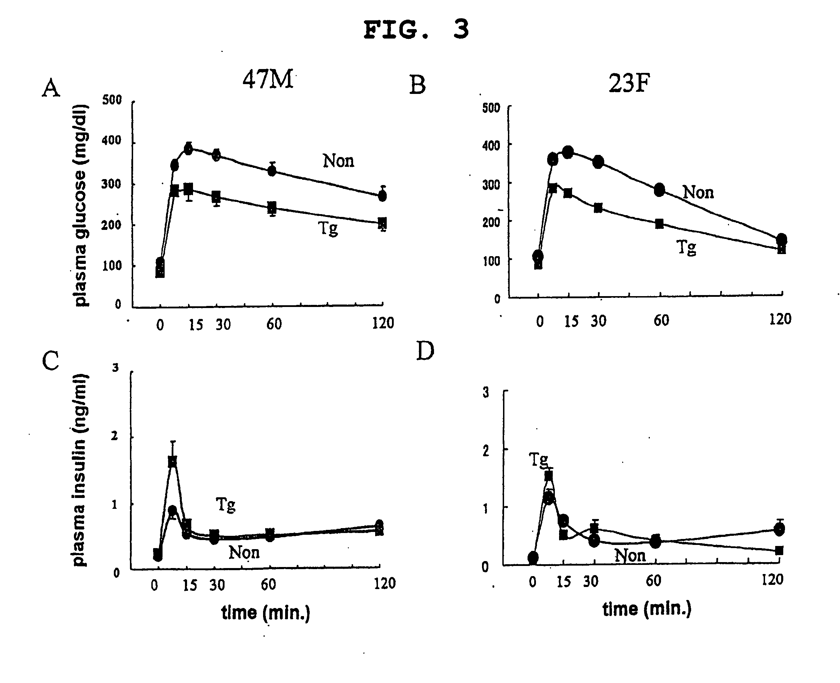

[FIG. 3] Graphs showing the results of a glucose tolerance test of transgenic mice. The glucose tolerance test was performed on 16-week-old 47M strain (A, C) mice and 18-week-old 23F strain (B, D) mice fed with a normal diet, after 16 hours of fasting. (A, B) show plasma glucose levels, (C, D) show plasma insulin levels (Mean±SE, 47M n=8; 23F n=12). NonTg: control mice, Tg: transgenic mice.

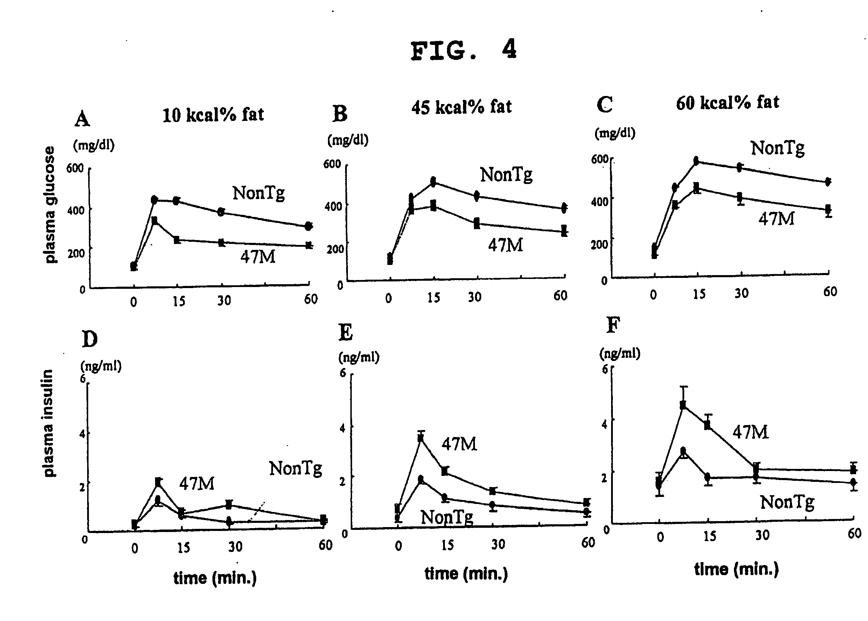

[FIG. 4] Graphs showing the results of a glucose tolerance test in fatty diet load groups of transgenic mice. The glucose tolerance test was performed on the transgenic mouse 47M strain, after 16 hours of fasting following loading of a low-fat diet with 10 kcal% fat (A, D), a high-fat diet with 45 kcal% fat (B, E), or a high-fat diet with 60 kcal% fat (C, F) for 9 weeks from 8 week-old. (A, B, C) show plasma glucose levels; (D, E, F) show plasma insulin levels (Mean±SE, n=10). NonTg: control mice, Tg: transgenic mouse 47M strain.

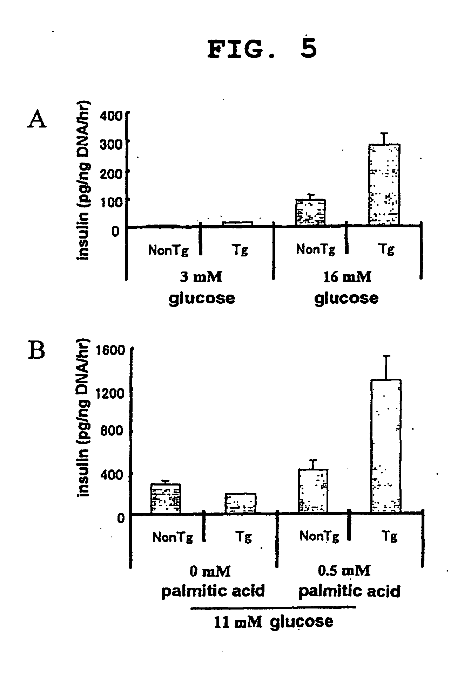

[FIG. 5] Graphs showing insulin secretion in transgenic mice upon glucose stimulation. (A) Shown is insulin secretion in the presence of 3 mM glucose or 16 mM glucose in the transgenic mouse 47M strain. (B) Shown is insulin secretion with or without addition of 0.5 mM palmitic acid in the presence of 11 mM glucose in the transgenic mouse 47M strain. The axis of ordinates indicates the amount of insulin secreted; each bar and error bar indicate a mean and a standard deviation, respectively (Mean±SE, n=3). NonTg: control mice, Tg: transgenic mouse 47M strain.

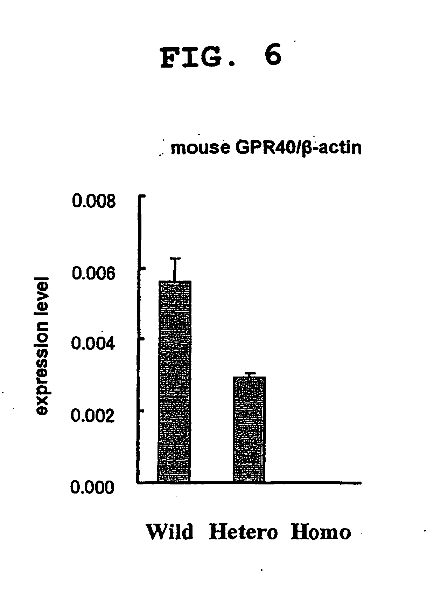

[FIG. 6] A graph showing the expression of the mouse GPR40 gene in GPR40 gene homo-deficient mice. The expression of the mouse GPR40 gene in the pancreas at 8 week-old is shown. The expression level of the mouse GPR40 gene is shown as a ratio to the expression level of the actin gene. Each bar and error bar indicate a mean and a standard deviation, respectively (Mean±SE, n=3). Wild: control mice, Hetero: GPR40 gene hetero-deficient mice, Homo: GPR40 gene homo-deficient mice.

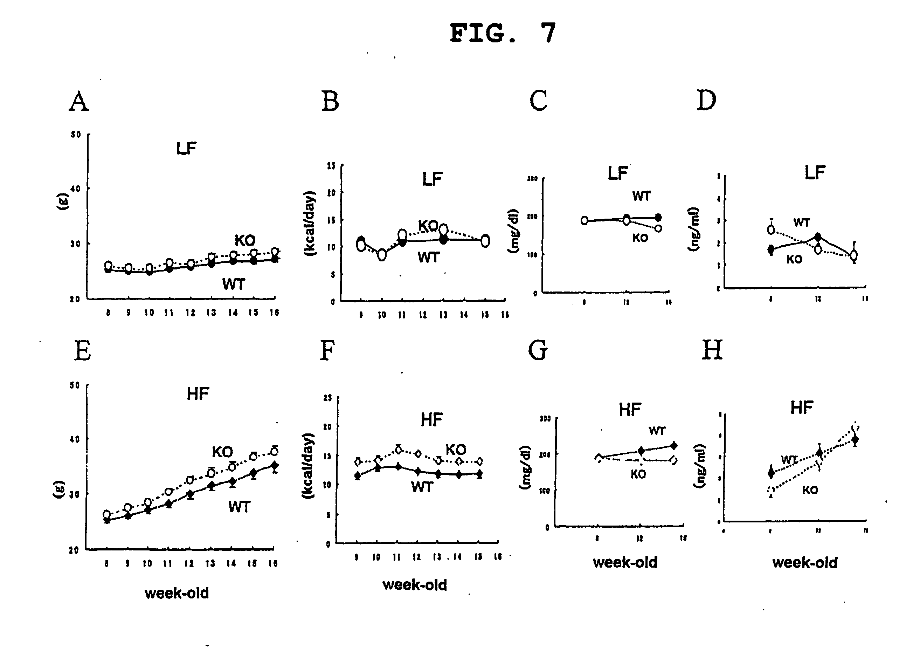

[FIG. 7] Graphs showing general properties in GPR40 gene homo-deficient mice. GPR40 gene homo-deficient mice were loaded with a low-fat diet with 10 kcal% fat (A, B, C, D) or a high-fat diet with 60 kcal% fat (E, F, G, H) for 8 weeks from 8 week-old. (A, E), (B, F), (C, G), and (D, H) show body weights, calorific intakes, plasma glucose levels, and plasma insulin levels, respectively (Mean±SE, n=10). WT: control mice, KO: GPR40 gene homo-deficient mice, LF: low-fat diet load group, HF: high-fat diet load group.

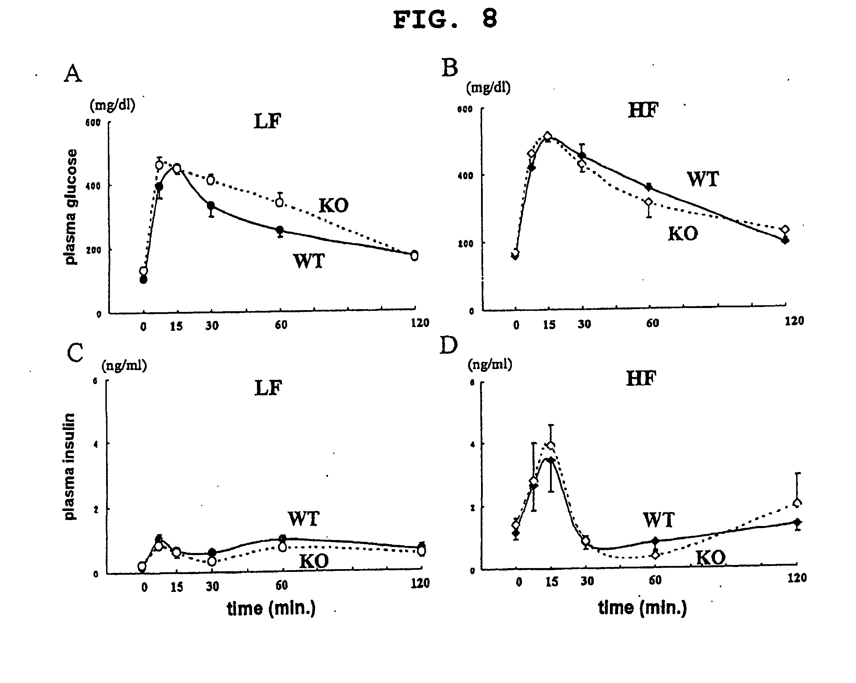

[FIG. 8] Graphs showing the results of a glucose tolerance test in fatty diet load groups of GPR40 gene homo-deficient mice. The glucose tolerance test was performed on GPR40 gene homo-deficient after 16 hours of fasting following loading with a low-fat diet with 10 kcal% fat (A, C) or a high-fat diet with 60 kcal% fat (B, D) for 11 weeks from 8 week-old. (A, B) show plasma glucose levels; (C, D) show plasma insulin levels (Mean±SE, n=8). WT: control mice, KO: GPR40 gene homo-deficient mice, LF: low-fat diet load group, HF: high-fat diet load group.

A transgenic mouse retaining a DNA that encodes an exogenous GPR40 in an expressible state (hereinafter sometimes referred to as "the Tg animal of the present invention") stably retains a DNA that encodes an exogenous GPR40 in an expressible state. "Stably retains" means that the DNA that encodes the exogenous GPR40 is permanently present in an expressible state in the cells of the animal; although the DNA may be integrated into a host chromosome, or may be stably present as an extra-chromosomal DNA, the DNA is preferably retained in an integrated state into a host chromosome.

The Tg animal of the present invention is prepared by introducing a desired DNA that encodes an exogenous GPR40 into a fertilized egg, unfertilized egg, spermatozoa, and precursor cell thereof (primordial germ cell, oogonium, oocyte, egg cell, spermatogonium, spermatocyte, spermatid and the like) and the like of a non-human mammal, preferably at an early stage of the embryogenesis of a fertilized egg (more preferably prior to the 8-cell stage), by a method of gene introduction such as the calcium phosphate co-precipitation method, electroporation method, lipofection method, aggregation method, microinjection method, gene gun (particle gun) method, or DEAE-dextran method. It is also possible to introduce a desired DNA into a somatic cell, tissue, organ and the like of a non-human mammal by the method of gene introduction, and to utilize them for cell culture, tissue culture and the like; furthermore, by fusing this cell with the above-described embryo (or germ) cell using a commonly known method of cell fusion, a Tg animal can also be prepared. Alternatively, as in the preparation of a knockout animal, by introducing a desired DNA into an embryonic stem cell (ES cell) of a non-human mammal with the above-described method of gene introduction, selecting a clone having the DNA integrated stably, and then injecting the ES cell into a blastocyst or allowing an ES cell mass and an 8-cell stage embryo to aggregate together, to prepare a chimera mouse, and selecting an animal having the introduced DNA transmitted to the germ line, a Tg animal can also be obtained.

A portion of the living body of a Tg animal prepared as described above (for example, (1) a cell, tissue, organ and the like that stably retain a DNA that encodes an exogenous GPR40, (2) a cell or tissue derived therefrom, in culture, passaged as required, and the like) can also be used as "a portion of the living body of a non-human mammal retaining a DNA that encodes an exogenous GPR40 in an expressible state" of the present invention for the same purpose as that of "a non-human mammal retaining a DNA that encodes an exogenous GPR40 in an expressible state" of the present invention.
Examples of preferable portions of the living body of the Tg animal of the present invention include organs such as the pancreas, liver, fat tissue, skeletal muscle, kidney, adrenal, blood vessel, heart, gastrointestinal tract, and brain, tissue pieces and cells and the like derived from the organs.

"A non-human mammal" that can be a subject of the present invention is a non-human mammal for which a transgenic system has been established, namely a mouse. In particular, from the viewpoint of the preparation of disease model animals, mice, which have relatively short period of ontogeny and life cycles, and which are easy to propagate (for example, C57BL/6 strain, DBA2 strain and the like as pure strains, B6C3F₁ strain, BDF, strain, B6D2F₁ strain, BALB/c strain, ICR strain and the like as hybrid strains) are preferable.

"A DNA that encodes an exogenous GPR40" is not particularly limited, as long as it is not an endogenous GPR40 DNA intrinsically present in the non-human mammal being the subject of gene introduction, but an externally introduced one; this may be a GPR40 DNA derived from the non-human mammal (for example, mouse) being the subject of gene introduction, or a DNA that encodes a GPR40 derived from a heterogeneous mammal (for example, humans, bovines, monkeys, pigs, sheep, goat, rabbits, dogs, cats, guinea pigs, hamsters, rats and the like) or a protein having substantially the same amino acid sequence thereas. Preferably, the DNA that encodes an exogenous GPR40 for use in the present invention is a DNA that encodes a GPR40 heterogeneous to the non-human mammal being the subject of gene introduction or a protein having substantially the same amino acid sequence thereas, more preferably a DNA that encodes human GPR40 or a protein having substantially the same amino acid sequence thereas. A DNA that encodes human GPR40 includes a DNA that encodes the amino acid sequence shown by SEQ ID NO:2, preferably a DNA comprising the base sequence shown by SEQ ID NO:1. "Substantially the same amino acid sequence" includes, in the case of human GPR40, for example, amino acid sequences having an identity of about 90% or more, preferably 95% or more, more preferably about 98% or more, to the amino acid sequence shown by SEQ ID NO:2, and the like. Amino acid sequence identity can be calculated using the homology calculating algorithm NCBI BLAST (National Center for Biotechnology Information Basic Local Alignment Search Tool) under the following conditions (expect=10; gap allowed; matrix=BLOSUM62; filtering=OFF).
"A protein having substantially the same amino acid sequence" is preferably, in the case of human GPR40, for example, a protein comprising substantially the same amino acid sequence as the amino acid sequence shown by SEQ ID NO:2, and having substantially the same quality of activity as the protein consisting of the amino acid sequence shown by SEQ ID NO:2. Examples of "substantially the same quality of activity" include ligand [i.e., free fatty acids and the like (including agonists and antagonists)] binding activity, intracellular Ca⁺⁺ concentration raising action, cAMP production suppressive action, promoting action on insulin secretion from pancreatic β cells and the like. Substantially the same quality means that these activities are qualitatively equivalent. Therefore, it is preferable that the proteins be equivalent to each other in terms of ligand binding activity, intracellular Ca⁺⁺ concentration raising action, cAMP production suppressive action, insulin secretion promoting action and the like, but quantitative factors such as the degrees of these activities (e.g., about 0.01 to 100 times, preferably about 0.5 to 20 times, more preferably about 0.5 to 2 times) and the molecular weight of the protein may be different. Measurements of activities such as ligand binding activity, intracellular Ca⁺⁺ concentration raising action, cAMP production suppressive action, and insulin secretion promoting action can be performed in accordance with methods known per se; for example, these activities can be measured by the screening methods described in patent document 3 above and the like.
DNAs that encode a GPR40 derived from a non-human mammal include the mouse-, rat- or hamster-derived DNAs described in patent document 3 above and the like.

The DNA that encodes an exogenous GPR40 is preferably in an intron-free form (i.e., complementary DNA) like, for example, a DNA comprising the base sequence shown by SEQ ID NO:1; however, in another embodiment, an intron-containing form (i.e., genomic DNA) can also be used preferably because the 5'- and 3'-terminal sequences of introns are common to most eukaryotic genes [however, because the ORF of human GPR40 (SEQ ID NO:1) and the ORF of mouse GPR40F (GenBank accession No. NM_194057.1) is consistent with the 40534295 position to 40535197 position of human chromosome 19 (GenBank accession No. NC_000019 (VERSION: NC_000019.8 GI:42406306)) and the 113965103 position to 113966005 position (reverse strand) of mouse chromosome 7 (GenBank accession No. NC_000073 (VERSION: NC_000073.3 GI:83280982)), respectively, no intron is present at least in the human and mouse GPR40 genes].

The DNA that encodes an exogenous GPR40 can be isolated by a hybridization method or PCR method and the like, using all or a portion of a DNA derived from the pancreas, liver, fat tissue, skeletal muscle, kidney, adrenal, blood vessel, heart, gastrointestinal tract, brain and the like of a human or various non-human mammals (bovines, monkeys, pigs, sheep, goat, rabbits, dogs, cats, guinea pigs, hamsters, rats, mice and the like) and a genomic DNA derived from a commercially available genomic DNA library as the starting material, or using a cDNA prepared by a commonly known method from an RNA derived from the pancreas, liver, fat tissue, skeletal muscle, kidney, adrenal, blood vessel, heart, gastrointestinal tract, brain and the like of a human or various non-human mammals as the starting material, using an oligonucleotide prepared on the basis of a commonly known GPR40 gene sequence as the probe or primer.

The Tg animal of the present invention retains a DNA that encodes an exogenous GPR40 "in an expressible state". Therefore, to introduce the DNA into a subject animal, it is generally advantageous to use the DNA in a form containing an expression cassette wherein the DNA is joined downstream of a promoter capable of functioning in the cells of the subject animal (e.g., expression vector and the like).

Useful vectors for carrying a DNA that encodes an exogenous GPR40 include Escherichia coli-derived plasmids, *Bacillus subtilis*-derived plasmids, yeast-derived plasmids, bacteriophages such as λ phage, animal or insect viruses such as retrovirus such as Moloney leukemia virus, lentivirus, adeno-associated virus, vaccinia virus and baculovirus, and the like. In particular, plasmids (preferably plasmids derived from *Escherichia coli, Bacillus subtilis*, or yeast, particularly plasmids derived from Escherichia coli) and animal viruses (preferably retrovirus, lentivirus) are preferable.

Examples of the promoter that regulates the expression of an exogenous GPR40 gene include promoters of genes derived from viruses (e.g., cytomegalovirus, Moloney leukemia virus, JC virus, breast cancer virus and the like), promoters of genes derived from various mammals (humans, bovines, monkeys, pigs, sheep, goat, rabbits, dogs, cats, guinea pigs, hamsters, rats, mice and the like) and birds (chicken and the like) [for example, albumin, endothelin, osteocarcine, muscular creatine kinase, type I and type II collagen, cyclic AMP-dependent protein kinase βI subunit, atrial natriuretic factor, dopamine β-hydroxylase, neurofilament light chain, metallothionein I and IIA, metalloproteinase 1 tissue inhibitor, smooth muscle α actin, polypeptide chain elongation factor 1α (EF1-α), β actin, α and β myosin heavy chains, myosin light chains 1 and 2, myelin basic protein, serum amyloid P component, renin and the like] and the like. Preferably, according to the desired disease model, promoters capable of specifically or highly expressing an exogenous GPR40 in the target tissue [e.g., insulin I and II promoters, which allow specific expression in the pancreas (hereinafter, also generically referred to as insulin promoters), or original promoters of GPR40; gene promoters of serum amyloid P component (SAP), albumin, transferrin, fibrinogen, antithrombin III, α1-antitrypsin and the like, which allow high expression in the liver; adipocytokine gene promoters of adiponectin and the like, which allow specific expression in fat tissue; gene promoters of musclin and the like, which allow specific expression in skeletal muscles; gene promoters of PTH/PTHrP receptor and the like, which allow high expression in the kidney; gene promoters of ACTH receptor and the like, which allow high expression in the adrenal; gene promoters of preproendothelin-1 and the like, which allow specific expression in blood vessels; gene promoters of α and P myosin heavy chains, myosin light chains 1 and 2 and the like, which allow high expression in the heart; gene promoters of fatty acid-binding proteins and the like, which allow high expression in the gastrointestinal tract; gene promoters of myelin basic protein, glial fibrillary acidic protein and the like, which allow high expression in the brain, and the like] can be chosen as appropriate; preferably, promoters capable of conferring a temporal and spatial expression profile similar to the expression profile of an endogenous GPR40 gene under physiological conditions, for example, insulin promoters, GPR40 promoters and the like, are used, and more preferably, insulin II promoter is used.

It is preferable that a sequence that terminates the transcription of the desired messenger RNA in the Tg animal (a polyadenylation (polyA) signal, also called a terminator) be present downstream of the DNA that encodes an exogenous GPR40; for example, using a terminator sequence derived from a virus gene, or derived from a gene of various mammals or birds, efficient expression of the transgene can be achieved. Preferably, the SV40 terminator of simian virus and the like are used. In addition, for the purpose of increasing the expression of the desired gene, the splicing signal of each gene, an enhancer region, or a portion of the intron of an eukaryotic gene can also be joined 5' upstream of the promoter region, between the promoter region and the coding region, or 3' downstream of the coding region, depending on the purpose.

When a Tg animal is prepared using an embryonic stem cell (ES cell), the above-described vector preferably further comprises a selection marker gene (e.g., drug resistance genes such as neomycin resistance gene and hygromycin resistance gene) for selecting a clone having the introduced DNA stably integrated therein. Furthermore, when it is intended to integrate the introduced DNA at a particular location on the host chromosome by homologous recombination (i.e., preparation of a knockin animal), the above-described vector preferably further comprises the herpes simplex virus-derived thymidine kinase (HSV-tk) gene or the diphtheria toxin gene as a negative selection marker gene outside a DNA sequence homologous to the target site, in order to avoid random insertions. These embodiments will be described in detail below.

The above-described promoter, DNA that encodes an exogenous GPR40, terminator and the like can be inserted into the above-described vector in the right arrangement, i.e., in an arrangement that allows the expression of the exogenous GPR40 in the Tg animal, by an ordinary gene engineering technique using an appropriate restriction enzyme and DNA ligase and the like.

In a preferred embodiment, the expression vector comprising a DNA that encodes an exogenous GPR40, obtained as described above, is introduced to an early embryo of a non-human mammal being the subject by microinjection.

An early embryo of the subject non-human mammal can be obtained by collecting an in vivo fertilized egg obtained by mating a male and female non-human mammal of the same species, or by in vitro fertilization of an ovum and spermatozoa respectively collected from a female and male non-human mammal of the same species.
The age, rearing conditions and the like of the non-human mammal used vary depending on animal species; for example, when a mouse (preferably, a mouse of an inbred strain such as C57BL/6J(B6), F₁ of B6 and another inbred strain, and the like) is used, it is preferable that a female at about 4 to about 6 week-old and a male at about 2 to about 8 month-old be used, and that the mice be reared with a light period of about 12 hours per day (for example, 7:00-19:00) for about 1 week.
Although the in vivo fertilization may be by natural mating, a method is preferable comprising administering a gonadotropic hormone to a female non-human mammal to induce overovulation, and then mating the female with a male non-human mammal, for the purpose of adjusting the estrous cycle and obtaining a large number of early embryos from a single individual. For inducing ovulation in a female non-human mammal, for example, a method is preferable comprising administering a follicle-stimulating hormone (pregnant mare serum gonadotropin, generally abbreviated as PMSG), and then a luteinizing hormone (human chorionic gonadotropin, generally abbreviated as hCG), by, for example, intraperitoneal injection and the like; preferable amounts and frequencies of administration of the hormones vary depending on the species of the non-human mammal. For example, when the non-human mammal is a mouse (preferably, a mouse of an inbred strain such as C57BL/6J(B6), F₁ of B6 and another inbred strain, and the like), generally a method is preferable comprising administering a follicle-stimulating hormone, then administering a luteinizing hormone about 48 hours later, and immediately mating the female mouse with a male mouse to obtain a fertilized egg, wherein the amount of the follicle-stimulating hormone administered is about 20 to about 50 IU/individual, preferably about 30 IU/individual, and the amount of the luteinizing hormone administered is about 0 to about 10 IU/individual, preferably about 5 IU/individual.
After elapse of a given time, a female non-human mammal confirmed to have copulated by vaginal plug examination and the like is laparotomized, a fertilized egg is removed from the oviduct, washed in a medium for embryo culture (e.g., M16 medium, modified Whitten medium, BWW medium, M2 medium, WM-HEPES medium, BWW-HEPES medium and the like) to remove cumulus oophorus cells, and cultured in 5% gaseous carbon dioxide/95% air by the microdrop culture method and the like until DNA microinjection. If microinjection is not immediately performed, the fertilized egg collected may be stored under freezing by the slow method or the ultrarapid method and the like.

Meanwhile, in the case of in vitro fertilization, a follicle-stimulating hormone and a luteinizing hormone are administered to a female non-human mammal for egg collection (the same as in in vivo fertilization is preferably used) as described above to induce ovulation, after which ova are collected and cultured in a medium for fertilization (e.g., TYH medium) in 5% gaseous carbon dioxide/95% air by the microdrop culture method and the like until in vitro fertilization. Separately, the cauda epididymidis is removed from a male non-human mammal of the same species (the same as in in vivo fertilization is preferably used), and a spermatozoa mass is collected and precultured in a medium for fertilization. After completion of the preculture, spermatozoa are added to the medium for fertilization containing the ova, and the ova are cultured in 5% gaseous carbon dioxide/95% air by the microdrop culture method and the like, after which a fertilized egg having two pronuclei is selected under a microscope. If DNA microinjection is not immediately performed, the fertilized egg obtained may be stored under freezing by the slow method or the ultrarapid method and the like.

DNA microinjection into the fertilized egg can be performed by a conventional method using a commonly known device such as a micromanipulator. Briefly, the fertilized egg placed in a microdrop of a medium for embryo culture is aspirated and immobilized using a holding pipette, and a DNA solution is injected directly into the male or female pronucleus, preferably into the male pronucleus, using an injection pipette. The introduced DNA is used preferably after being highly purified using CsCl density gradient ultracentrifugation or an anion exchange resin column and the like. It is also preferable that the introduced DNA be linearized in advance by cutting the vector portion using a restriction enzyme.

After introducing the DNA, the fertilized egg is cultured in a medium for embryo culture in 5% gaseous carbon dioxide/95% air by the microdrop culture method and the like until the 1-cell stage to blastocyst stage, after which it is transplanted to the oviduct or uterus of a pseudopregnant embryo recipient female non-human mammal. The embryo recipient female non-human mammal for embryo reception may be any one of the same species as the animal from which the early embryo to be transplanted is derived; for example, when a mouse early embryo is transplanted, a female ICR mouse (preferably about 8 to about 10 week-old) and the like are preferably used. A known method of rendering an embryo recipient female non-human mammal pseudopregnant is, for example, a method comprising mating the female with a vasectomized (vasoligated) male non-human mammal of the same species (for example, in the case of a mouse, with a male ICR mouse (preferably about 2 month-old or more)), and selecting a female confirmed to have a vaginal plug.
The embryo recipient female used may be one that has ovulated naturally, or one receiving luteinizing hormone releasing hormone (generally abbreviated LHRH) or an analogue thereof administered prior to mating with a vasectomized (vasoligated) male, to induce fertility. Examples of the LHRH analogue include [3,5-DiI-Tyr⁵]-LH-RH, [Gln⁸]-LH-RH, [D-Ala⁶]-LH-RH, [des-Gly¹⁰]-LH-RH, [D-His(Bzl)⁶]-LH-RH and Ethylamides thereof and the like. The amount of LHRH or an analogue thereof administered, and the timing of mating with a male non-human mammal after the administration vary depending on the species of the non-human mammal. For example, when the non-human mammal is a mouse (preferably an ICR mouse and the like), it is usually preferable that the female mouse be mated with a male mouse about 4 days after administration of LHRH or an analogue thereof; the amount of LHRH or an analogue thereof administered is usually about 10 to 60 µg/individual, preferably about 40 µg/individual.

Usually, if the early embryo to be transplanted is in the morula stage or after, the embryo is transplanted to the uterus of an embryo recipient female; if the early embryo is in a stage before the morula stage (for example, 1-cell stage to 8-cell stage embryo), the embryo is transplanted to the oviduct. The female for embryo reception is used as appropriate after elapse of a given number of days after becoming pseudopregnant depending on the developmental stage of the embryo to be transplanted. For example, in the case of a mouse, a female mouse at about 0.5 days after becoming pseudopregnant is preferable for the transplantation of a 2-cell stage embryo, and a female mouse at about 2.5 days after becoming pseudopregnant is preferable for the transplantation of a blastocystic embryo. After the embryo recipient female is anesthetized (preferably, Avertin, Nembutal and the like are used), an incision is made, the ovary is pulled out, and early embryos (about 5 to about 10 embryos) in suspension in a medium for embryo culture are injected into the vicinity of the abdominal osteum of the uterine tube or the uterine tube junction of the uterine horn using a pipette for embryo transplantation.

If the transplanted embryo implants successfully and the embryo recipient female becomes pregnant, non-human mammal pups will be obtained by natural delivery or caesarean section. Embryo recipient females that have delivered naturally are allowed to continue suckling; if the pups are delivered by caesarean section, the pups can be suckled by a separately provided female for suckling (for example, in the case of the mouse, a female mouse with usual mating and delivery (preferably a female ICR mouse and the like)).

Introduction of the DNA that encodes an exogenous GPR40 at the fertilized egg cell stage is secured so that the introduced DNA will be present in all of the germline cells and somatic cells of the subject non-human mammal. Whether or not the introduced DNA is integrated into chromosome DNA can be determined by, for example, screening chromosome DNAs separated and extracted from the tail of the pup, by Southern hybridization or PCR. The presence of a DNA that encodes an exogenous GPR40 in the germline cells of the offspring non-human mammal (F₀) obtained as described above means that the DNA that encodes the exogenous GPR40 is present in all of the germline cells and somatic cells of all animals in the subsequent generation (F₁).
Usually, F₀ animals are obtained as heterozygotes having the introduced DNA in either one of the homologous chromosomes. Different F₀ individuals have the introduced DNA inserted randomly into different chromosomes unless the insertion is by homologous recombination. To obtain a homozygote having the DNA that encodes the exogenous GPR40 in both of the homologous chromosomes, an F₀ animal and a non-transgenic animal are crossed to prepare an F₁ animal, and heterozygous siblings thereof having the introduced DNA in either one of the homologous chromosomes may be crossed. If the introduced DNA is integrated only at one gene locus, 1/4 of the F₂ animals obtained will be homozygotes.

In another embodiment, an expression vector comprising a DNA that encodes an exogenous GPR40 is introduced into an ES cell of the non-human mammal being the subject by a commonly known method of gene introduction such as electroporation.

An ES cell refers to a cell derived from an inner cell mass (ICM) of a fertilized egg in the blastocyst stage, and can be cultivated and maintained while keeping the undifferentiated state in vitro. ICM cells are destined to form the embryo body, being stem cells on which all tissues, including germ cells, are based. The ES cell used may be of an established cell line, or of a cell line newly established in accordance with the method of Evans and Kaufman (Nature, vol.292, p.154, 1981). For example, in the case of mouse ES cells, ES cells derived from a 129 strain mouse are currently generally used, but the immunological background thereof is unclear; for the purposes of acquiring ES cells of a pure strain instead thereof with an immunologically clear genetic background and the like, an ES cell established from a C57BL/6 mouse or from a BDF₁ mouse (F₁ of C57BL/6 and DBA/2), wherein the small number of ova collectable from C57BL/6 has been improved by crossing with DBA/2, and the like can also be used suitably. In addition to being advantageous in that the number of ova collectable is high, and that the ova are robust, BDF₁ mice have the C57BL/6 mouse as the background thereof; therefore, ES cells derived therefrom can be used advantageously in that, when preparing a disease model mouse, the genetic background can be replaced with that of the C57BL/6 mouse by back-crossing with a C57BL/6 mouse.

ES cells can be prepared by, for example, as described below. When a blastocystic embryo is collected from the uterus of a female non-human mammal [for example, when a mouse (preferably a mouse of an inbred strain such as C57BL/6J(B6), F₁ of B6 and another inbred strain, and the like) is used, a female mouse at about 8 to about 10 week-old (about 3.5 days of gestation) mated with a male mouse at about 2 month-old or more is preferably used] (or an early embryo in the morula stage or before is collected from the oviduct, after which it may be cultured in a medium for embryo culture as described above until the blastocyst stage), and cultured on a layer of appropriate feeder cells (for example, in the case of a mouse, primary fibroblasts prepared from a fetal mouse, commonly known STO fibroblast line and the like), some cells of the blastocyst gather to form an ICM that will differentiate into an embryo. This inner cell mass is trypsinized to dissociate single cells, and while maintaining an appropriate cell density and making medium exchanges, dissociation and passage are repeated, whereby ES cells are obtained.

Although both male and female ES cells can be used, male ES cells are usually more convenient in preparing a germline chimera. Also for the sake of saving painstaking labor for cultivation, it is desirable that sex identification be performed as early as possible. An example of the method of identifying the sex of an ES cell is a method comprising amplifying and detecting a gene in the sex determining region on Y chromosome by PCR. Using this method, about 1 colony of ES cells (about 50 cells) is sufficient, compared with the conventional method, which requires about 10⁶ cells for karyotype analysis, so that primary selection of ES cells in early stages of cultivation can be performed by sex identification, thus making early selection of male cells possible, whereby labor in early stages of cultivation can be reduced significantly.
Secondary selection can be performed by, for example, confirming chromosome numbers by the G-banding method, and the like. It is desirable that the chromosome number of the ES cell obtained be 100% of the normal number; however, if this is difficult to achieve because of physical operations in establishing the cell line and the like, it is desirable that after gene introduction into the ES cell, the gene be recloned into a normal cell (for example, in the case of a mouse, a cell whose chromosome number is 2n=40).

The ES cell line thus obtained needs to be subcultured carefully to maintain the nature of undifferentiated stem cells. For example, the ES cell line is cultured by, for example, a method comprising culturing on appropriate feeder cells, like STO fibroblasts, in the presence of LIF (1 to 10,000 U/ml), known as a differentiation suppressing factor, in a gaseous carbon dioxide incubator (preferably, 5% gaseous carbon dioxide/95% air or 5% oxygen/5% gaseous carbon dioxide/90% air) at about 37°C, and the like; upon passage, for example, the ES cell line is treated with trypsin/EDTA solution (usually 0.001 to 0.5% trypsin/0.1 to 5 mM EDTA, preferably about 0.1% trypsin/1 mM EDTA) to obtain single cells, which are sown onto freshly prepared feeder cells, and the like. This passage is normally performed every 1 to 3 days, during which the cells were examined; if a morphologically abnormal cell is found, it is desirable that the cultured cells be discarded.
ES cells can be differentiated into a wide variety of types of cell, including parietal muscle, visceral muscles, and cardiac muscle, by monolayer culture until the reach of a high density, or suspension culture until the formation of cell aggregates, under appropriate conditions [M.J. Evans and M.H. Kaufman, Nature vol.292, p.154, 1981; G.R. Martin, Proceedings of the National Academy of Sciences, USA (Proc. Natl. Acad. Sci. U.S.A.), vol.78, p.7634, 1981; T. C. Doetschman et al., Journal of Embryology and Experimental Morphology, vol.87, p.27, 1985]; the non-human mammal cells expressing an exogenous GPR40, according to the present invention, obtained by differentiating an ES cell incorporating a DNA that encodes an exogenous GPR40, are useful in cell biological investigations of exogenous GPR40 in vitro.

Although any of the calcium phosphate co-precipitation method, electroporation method, lipofection method, retrovirus infection method, aggregation method, microinjection method, gene gun (particle gun) method, DEAE-dextran method and the like can be used for gene introduction into ES cells, the electroporation method is generally chosen because of the ease of treatment of a large number of cells and the like. For the electroporation, ordinary conditions used for gene introduction into animal cells may be used as is; for example, the electroporation can be performed by trypsinizing ES cells in the logarithmic growth phase to disperse them as single cells, suspending the cells in a medium to obtain a density of 10⁶ to 10⁸ cells/ml, transferring the cells to a cuvette, adding 10 to 100 µg of a vector comprising a DNA that encodes an exogenous GPR40, and applying an electric pulse of 200 to 600 V/cm.

ES cells having the introduced DNA integrated therein can be determined by screening chromosome DNA separated and extracted from a colony obtained by culturing the single cells on feeder cells, by Southern hybridization or PCR; the biggest feature of transgenic systems using ES cells resides in the fact that transformants can be selected at the cell level with the expression of a drug resistance gene or a reporter gene as the index. Therefore, the introduction vector used here desirably further comprises, in addition to an expression cassette comprising a DNA that encodes an exogenous GPR40, a selection marker gene such as a drug resistance gene (e.g., neomycin phosphotransferase II (nptII) gene, hygromycin phosphotransferase (hpt) gene and the like) or a reporter gene (e.g., β-galactosidase (lacZ) gene, chloramphenicol acetyltransferase (cat) gene and the like). For example, when a vector comprising the nptII gene as the selection marker gene is used, ES cells after gene introduction treatment are cultured in a medium containing a neomycin-series antibiotic such as G418, the resulting resistant colonies are transferred to respective culture plates, and trypsinization and medium exchanges are repeated, after which a portion is reserved for cultivation, and the remainder is subjected to PCR or Southern hybridization to confirm the presence of the introduced DNA.

When an ES cell confirmed to have the introduced DNA integrated therein is returned to an embryo derived from a non-human mammal of the same species, the ES cell gets integrated into the ICM of the host embryo to form a chimeric embryo. This is transplanted into a recipient mother (embryo recipient female) and allowed to continue development, whereby a chimeric transgenic animal is obtained. If the ES cell contributes to the formation of a primordial germ cell that will differentiate into an egg or spermatozoon in the chimera animal, a germline chimera will be obtained; by mating this, a Tg animal having the introduced DNA maintained genetically therein can be prepared.

For preparing a chimeric embryo, there are a method wherein early embryos up to the morula stage are adhered and aggregated together (aggregation chimera method) and a method wherein a cell is micro-injected into a blastocoel cavity of a blastocyst (injection chimera method). Although the latter has traditionally been widely conducted in the preparation of a chimeric embryo using an ES cell, a method wherein an aggregation chimera is created by injecting an ES cell into the zona pellucida of an 8-cell stage embryo, and a method wherein an aggregation chimera is created by co-culturing and aggregating an ES cell mass and an 8-cell stage embryo deprived of the zona pellucida, as a method which does not require a micromanipulator and which can be easily operated, have recently been conducted.
In all cases, a host embryo can be collected from a non-human mammal that can be used as a female for egg collection in gene introduction into a fertilized egg in the same manner; for example, in the case of a mouse, to make it possible to determine the percent contribution of ES cells to the formation of a chimera mouse by coat color, it is preferable that the host embryo be collected from a mouse of a strain showing a coat color different from that of the strain from which the ES cell is derived. For example, in the case of an ES cell derived from a 129 strain mouse (coat color: agouti), a C57BL/6 mouse (coat color: black) or an ICR mouse (coat color: albino) is used as the female for egg collection; in the case of an ES cell derived from a C57BL/6 or DBF₁ mouse (coat color: black) or from a TT2 cell (derived from F₁ (coat color: agouti) of C57BL/6 and CBA), an ICR mouse or a BALB/c mouse (coat color: albino) can be used as the female for egg collection.
Because the germline chimera formation capacity depends largely on the combination of an ES cell and a host embryo, it is more preferable that a combination showing a high germline chimera formation capacity be chosen. For example, in the case of a mouse, it is preferable to use a host embryo derived from the C57BL/6 strain and the like for ES cells derived from the 129 strain, and to use a host embryo derived from the BALB/c strain and the like for ES cells derived from the C57BL/6 strain.
It is preferable that the female mouse for egg collection be about 4 to about 6 week-old, and that the male mouse for mating be of the same strain at about 2 to about 8 month-old. Although the mating may be by natural mating, it is preferably performed after administering gonadotropic hormones (follicle-stimulating hormone, then luteinizing hormone) to induce overovulation.

In the case of the blastodisk injection method, a blastocystic embryo (for example, in the case of a mouse, at about 3.5 days after mating) is collected from the uterus of a female for egg collection (or an early embryo in the morula stage or before, after being collected from the oviduct, may be cultured in the above-described medium for embryo culture until the blastocyst stage), and ES cells (about 10 to about 15 cells) having a DNA that encodes an exogenous GPR40 introduced thereinto are injected into a blastocoel cavity of the blastocyst using a micromanipulator, after which the embryos are transplanted into the uterus of a pseudopregnant embryo recipient female non-human mammal. As the embryo recipient female non-human mammal, a non-human mammal that can be used as an embryo recipient female in gene introduction into a fertilized egg can be used in the same manner.
In the case of the co-culture method, 8-cell stage embryos and morulas (for example, in the case of a mouse, about 2.5 days after mating) are collected from the oviduct and uterus of a female for egg collection (or an early embryo in the 8-cell stage or before, after being collected from the oviduct, may be cultured in the above-described medium for embryo culture until the 8-cell stage or morula stage), and the zona pellucida is lysed in acidic Tyrode's solution, after which an ES cell mass incorporating a DNA that encodes an exogenous GPR40 (number of cells: about 10 to about 15 cells) is placed in a microdrop of a medium for embryo culture overlaid with mineral oil, the above-described 8-cell stage embryo or morula (preferably 2 embryos) is further placed, and they are co-cultured overnight. The morula or blastocyst obtained is transplanted to the uterus of an embryo recipient female non-human mammal as described above.

If the transplanted embryo implants successfully and the embryo recipient female becomes pregnant, chimeric non-human mammal pups will be obtained by natural delivery or caesarean section. Embryo recipient females that have delivered spontaneously are allowed to continue suckling; if the pups are delivered by caesarean section, the pups can be suckled by a separately provided female for suckling (a female non-human mammal with usual mating and delivery).
For the selection of a germline chimera, if the sex of the ES cell has already been determined, a chimera mouse of the same sex as the ES cell first is selected (usually, a male chimera mouse is chosen since a male ES cell is used), and then a chimera mouse showing a high ES cell contribution rate (for example, 50% or more) is selected on the basis of phenotypes such as coat color. For example, in the case of a chimera mouse obtained from a chimera embryo between a D3 cell, which is a male ES cell derived from a 129 strain mouse, and a host embryo derived from a C57BL/6 mouse, it is preferable that a male mouse showing a high percentage of the agouti coat color be selected. Whether or not the selected chimera non-human mammal is a germline chimera can be determined on the basis of the phenotypes of the F₁ animal obtained by crossing with an appropriate strain of the same animal species. For example, in the case of the above-described chimera mouse, agouti is dominant over black; therefore, when the male mouse is crossed with a female C57BL/6 mouse, the coat color of the F₁ obtained is agouti if the selected male mouse is a germline chimera.

The thus-obtained germline chimera non-human mammal incorporating a DNA that encodes an exogenous GPR40 (founder) is usually obtained as a heterozygote having the introduced DNA in either one of the homologous chromosomes. Individual founders have the introduced DNA inserted randomly into different chromosomes unless the insertion is by homologous recombination. To obtain a homozygote having a DNA that encodes an exogenous GPR40 in both homologous chromosomes, of the F₁ animals obtained as described above, siblings of heterozygotes having the introduced DNA in either one of the homologous chromosomes may be crossed. Selection of heterozygotes can be determined by, for example, screening chromosome DNAs separated and extracted from the tail of an F₁ animal by Southern hybridization or PCR. If the introduced DNA is integrated only at one gene locus, 1/4 of the F₂ animals obtained will be homozygotes.

Another preferred embodiment with the use of a virus as the expression vector is a method comprising infecting an early embryo or ES cell of a non-human mammal with a virus comprising a DNA that encodes an exogenous GPR40 (see, for example, Proceedings of the National Academy of Sciences, USA (Proc. Natl. Acad. Sci. USA), vol.99, No.4, pp. 2140-2145, 2002). For example, when retrovirus or lentivirus is used, cells (fertilized eggs preferably deprived of the zona pellucida) are sown to an appropriate incubator such as a culture dish, a virus vector is added to the culture broth (if desired, polybrene may be co-present), the cells are cultured for 1 to 2 days, after which, in the case of an early embryo, the embryo is transferred to the oviduct or uterus of a pseudopregnant embryo recipient female non-human mammal as described above, or in the case of an ES cell, a selection drug such as G418 or hygromycin is added and cultivation is continued as described above, and cells having the vector integrated therein are selected.
Furthermore, as described in the Proceedings of the National Academy of Sciences, USA (Proc. Natl. Acad. Sci. USA), vol.98, pp. 13090-13095, 2001, a spermatogonium collected from a male non-human mammal is infected with a virus vector during co-cultivation with STO feeder cells, after which the spermatogonium is injected into the seminiferous tube of a male infertile non-human mammal, and the male infertile non-human mammal is mated with a female non-human mammal, whereby exogenous GPR40 hetero-Tg (+/-) pups can be obtained efficiently.

The Tg animal of the present invention has the following characteristics:
(1) the insulin secretion capacity has been increased, and/or
(2) the glucose tolerance has been improved, compared with the corresponding non-transgenic animal.
Conventionally commonly known GPR40 Tg mice (see nonpatent document 3, patent document 4) develop diabetes mellitus accompanied by impaired insulin secretion. However, the above-described phenotypes of the Tg animal of the present invention support the concept that a GPR40 agonist promotes insulin secretion from pancreatic β cells and is useful as a prophylactic/therapeutic drug for diabetes mellitus. Specifically, in the Tg animal of the present invention, it is thought that because the DNA that encodes an exogenous GPR40 is under the control of an insulin promoter, the intrinsic functions of β cell have been enhnaced. Because the phenotypes of the Tg animal of the present invention were observed in two strains as described in an Example, the phenotypes are judged to be an effect based on the transgene, supporting the reflection of the normal GPR40 functions. In contrast, the commonly known GPR40 Tg mouse has the GPR40 expressed by the Ipf-1 promoter, different from the promoter used in the Tg animal of the present invention; therefore, it is thought that the different phenotypes were observed because of the difference in expression site, expression timing, expression level, or position of insertion in the chromosome, or any other reason.

The Tg animal of the present invention not only demonstrates the concept of preventing/treating diabetes mellitus with a GPR40 agonists, as described above, but also can be used in evaluation systems for GPR40 regulatory drugs (including agonists and antagonists) and the like.
Accordingly, the present invention also provides a screening method for a GPR40 agonist or a GPR40 antagonist, comprising applying a test substance to the Tg animal of the present invention or a portion of the living body thereof, and determining the GPR40 agonist activity or GPR40 antagonist activity. If a human type gene is expressed, the screening method of the present invention will be useful in evaluating compounds that act only on the human type gene.
Here, "agonist activity" refers to the nature of binding specifically to GPR40 to shift the equilibrium between the active form and inactive form of GPR40 toward active, the extent of which is not particularly limited. Therefore, "substances having agonist activity (agonists)" include what is called full agonists, as well as partial agonists. Meanwhile, "antagonist activity" refers to the nature of binding antagonistically to the ligand binding site of GPR40, but having no or almost no effect on the equilibrium between the active form and the inactive form, or the nature of binding to an any site of GPR40 to shift the equilibrium between the active form and inactive form of GPR40 toward inactive. Therefore, as used herein, "a substance having antagonist activity (antagonist)" is to be defined as a concept encompassing both what is called neutral antagonists and inverse agonists.

Specifically, in the screening method of the present invention, a test substance is administered to the Tg animal of the present invention. Useful test substances include, in addition to commonly known synthetic compounds, peptides, proteins, DNA libraries and the like, for example, tissue extracts, cell culture supernatants and the like of mammals (for example, mice, rats, pigs, bovines, sheep, monkeys, humans and the like). The GPR40 agonist/antagonist activity of a test substance can be determined with, for example, ligand (i.e., free fatty acids and the like) binding activity, intracellular Ca⁺⁺ concentration raising action, cAMP production suppressive action, promoting action on insulin secretion from pancreatic β cells or the like as the index. These can be measured in accordance with methods known per se.

The GPR40 agonist thus selected is useful as a prophylactic/therapeutic agent for diseases such as diabetes mellitus (type I and type II), glucose intolerance, hyperlipemia, and metabolic syndrome, pancreas function regulator (e.g., pancreas function improving agent), insulin secretion promoter, hypoglycemic agent, and pancreatic P cell protecting agent which are safe and of low toxicity in mammals. In addition, the GPR40 agonist can also be used as a prophylactic/therapeutic agent for diseases such as ketosis, acidosis, diabetic neuropathy, diabetic nephropathy, diabetic retinitis, arteriosclerosis, sexual dysfunction, dermatological disease, arthropathy, osteopenia, thrombotic disease, maldigestion, and memory and learning disturbance.

The GPR40 agonist can, for example, be used orally as tablets coated with sugar as required, capsules, elixirs, microcapsules and the like, or can be used parenterally in the form of an injection such as a sterile solution or suspension in water or another pharmaceutically acceptable liquid. The agonist can be prepared as a pharmaceutical preparation by being blended with a physiologically acceptable carrier, flavoring agent, excipient, vehicle, antiseptic, stabilizer, binder and the like, in a unit dosage form required for generally accepted preparation design. The amounts of active ingredients in these preparations are chosen as appropriate in consideration of the doses described below.

Examples of additives that can be blended in tablets, capsules and the like include binders such as gelatin, cornstarch, tragacanth and acacia, excipients such as crystalline cellulose, swelling agents such as cornstarch, gelatin, and alginic acid, lubricants such as magnesium stearate, sweeteners such as sucrose, lactose and saccharin, flavoring agents such as peppermint, acamono oil and cherry, and the like. When the formulation unit form is a capsule, it can further contain a liquid carrier like an oil or fat in addition to the above-described types of material. A sterile composition for injection can be formulated according to an ordinary preparation design such as dissolving or suspending an active substance, a naturally produced vegetable oil such as sesame oil or coconut oil, and the like in a vehicle like water for injection.

Examples of aqueous liquids for injection include isotonic solutions comprising physiological saline, glucose or another auxiliary drug (for example, D-sorbitol, D-mannitol, sodium chloride and the like) and the like, which may be used in combination with an appropriate solubilizer, for example, an alcohol (for example, ethanol and the like), a polyalcohol (for example, propylene glycol, polyethylene glycol and the like), a non-ionic surfactant (for example, polysorbate 80^{™}, HCO-50 and the like) and the like. Examples of oily liquids include sesame oil, soybean oil and the like, which may be used in combination with solubilizers benzyl benzoate, benzyl alcohol and the like. Also, aqueous liquids for injection may be blended with, for example, a buffering agent (for example, phosphate buffer solution, sodium acetate buffer solution and the like), a soothing agent (for example, benzalkonium chloride, procaine hydrochloride and the like), a stabilizer (for example, human serum albumin, polyethylene glycol and the like), a preservative (for example, benzyl alcohol, phenol and the like), an antioxidant and the like. The prepared injection liquid is ordinally filled in an appropriate ampoule.

Because the preparation thus obtained is safe and of low toxicity, it can be administered to, for example, mammals (for example, human, rats, mice, guinea pigs, rabbits, sheep, pigs, bovines, horses, cats, dogs, monkeys and the like), preferably to animals from which the exogenous GPR40 is derived (preferably humans).
The dose of the GPR40 agonist varies depending on the target disease, subject of administration, route of administration and the like; for example, in the case of oral administration for treatment of diabetes mellitus, the usual dosage for an adult (weighing 60 kg) is about 0.1 mg to about 100 mg, preferably about 1.0 to about 50 mg, more preferably about 1.0 to about 20 mg, per day. In the case of parenteral administration, the dose of the agonist varies depending on the subject of administration, target disease and the like; for example, in the case of administration as an injection to an adult (weighing 60 kg) for treatment of diabetes mellitus, the dose is about 0.01 to about 30 mg, preferably about 0.1 to about 20 mg, more preferably about 0.1 to about 10 mg, per day. If the subject of administration is a non-human animal, an amount converted per 60 kg of body weight can be administered.

The GPR40 antagonist selected by the screening method of the present invention is useful as a prophylactic/therapeutic agent for diseases such as obesity, hyperlipemia, type II diabetes mellitus, and insulin resistance syndrome, pancreas function regulator (e.g., pancreas function improving agent), insulin secretion suppressant, and hyperglycemic agent which are safe and of low toxicity in mammals (preferably animals from which the exogenous GPR40 is derived, more preferably humans). In addition, the GPR40 antagonist can also be used as a prophylactic/therapeutic agent for diseases such as hypoglycemia, hypertension, diabetic neuropathy, diabetic nephropathy, diabetic retinitis, edema, brittle diabetes mellitus, fat atrophy, insulin allergy, insulinoma, arteriosclerosis, thrombotic disease, fat toxicity, and cancers. The GPR40 antagonist can be prepared as a pharmaceutical preparation in the same manner as with the above-described GPR40 agonist, and can be administered orally or parenterally to mammals (for example, humans, rats, mice, guinea pigs, rabbits, sheep, pigs, bovines, horses, cats, dogs, monkeys and the like).

The dose of the GPR40 antagonist varies depending on the target disease, subject of administration, route of administration and the like; for example, in the case of oral administration for treatment of obesity, the usual dosage for an adult (weighing 60 kg) is about 0.1 mg to about 100 mg, preferably about 1.0 to about 50 mg, more preferably about 1.0 to about 20 mg, per day. In the case of parenteral administration, the dose of the antagonist varies depending on the subject of administration, target disease and the like; for example, in the case of administration as an injection to an adult (weighing 60 kg) for treatment of obesity, the dose is about 0.01 to about 30 mg, preferably about 0.1 to about 20 mg, more preferably about 0.1 to about 10 mg, per day. If the subject of administration is a non-human animal, an amount converted per 60 kg of body weight can be administered.

As described above, the Tg animal of the present invention has the following characteristics:
(1) the insulin secretion capacity has been increased, and/or
(2) the glucose tolerance has been improved,
compared with the corresponding non-transgenic animal; therefore, the animal is particularly useful in screening for (1) insulin secretion and/or (2) glucose tolerance regulatory drugs.

Accordingly, the present invention also provides a screening method for (1) insulin secretion and/or (2) glucose tolerance regulatory drugs, comprising applying a test substance to the Tg animal of the present invention or a portion of the living body thereof, and measuring the (1) insulin secretion and/or (2) glucose tolerance.

Specifically, in the screening method, a test substance is administered to the Tg animal of the present invention. Useful test substances include, in addition to commonly known synthetic compounds, peptides, proteins, DNA libraries and the like, for example, tissue extracts, cell culture supernatants and the like of mammals (for example, mice, rats, pigs, bovines, sheep, monkeys, humans and the like). The insulin secretion regulatory action and glucose tolerance regulatory action of a test substance can be measured by respective methods known per se, for example, the methods used in an Example below and the like.

The insulin secretion/glucose tolerance regulatory drug selected by the screening method can be used to improve insulin secretion and/or glucose tolerance in mammals (preferably animals from which the exogenous GPR40 is derived, more preferably humans). The regulatory drug can be prepared as a pharmaceutical preparation in the same manner as with the above-described GPR40 agonist, and can be administered orally or parenterally to mammals (for example, humans, rats, mice, guinea pigs, rabbits, sheep, pigs, bovines, horses, cats, dogs, monkeys and the like).

The dose of the insulin secretion/glucose tolerance regulatory drug varies depending on the target disease, subject of administration, route of administration and the like; for example, in the case of oral administration for treatment of diabetes mellitus, the usual dosage for an adult (weighing 60 kg) is about 0.1 mg to about 100 mg, preferably about 1.0 to about 50 mg, more preferably about 1.0 to about 20 mg, per day. In the case of parenteral administration, the dose of the regulatory drug varies depending on the subject of administration, target disease and the like; for example, in the case of administration as an injection to an adult (weighing 60 kg) for treatment of diabetes mellitus, the dose is about 0.01 to about 30 mg, preferably about 0.1 to about 20 mg, more preferably about 0.1 to about 10 mg, per day. If the subject of administration is a non-human animal, an amount converted per 60 kg of body weight can be administered.

A non-human mammal deficient in the expression of the GPR40 gene means a non-human mammal having the expression of the endogenous GPR40 inactivated, including not only GPR40 KO animals prepared from an ES cell having the GPR40 gene knocked out (KO) as described above, but also knockdown (KD) animals having the expression of the GPR40 gene inactivated by antisense or RNAi technology and the like. Here, "knockout (KO)" means making the production of complete mRNA impossible by destroying or removing the endogenous gene, whereas "knockdown (KD)" means inhibiting the translation from mRNA to protein to thereby inactivate the expression of the endogenous gene.

In the GPR40 gene KO/KD animal (hereinafter, also simply referred to as "the KO/KD animal"), the "non-human mammal" that can be the subject is not particularly limited, as long as it is a non-human mammal for which a transgenic system has been established; examples include, mice, rats, bovines, monkeys, pigs, sheep, goat, rabbits, dogs, cats, guinea pigs, hamsters and the like. Mice, rats, rabbits, dogs, cats, guinea pigs, hamsters and the like are preferable; in particular, from the viewpoint of the preparation of disease model animals, rodents, which have relatively short period of ontogeny and life cycles, and which are easy to propagate, are more preferable, and mice (for example, C57BL/6 strain, DBA2 strain and the like as pure strains, and B6C3F₁ strain, BDF₁ strain, B6D2F₁ strain, BALB/c strain, ICR strain and the like as hybrid strains) and rats (for example, Wistar, SD and the like) are particularly preferable.

Regarding specific means for knocking out the GPR40 gene, it is preferable to use a method comprising isolating the GPR40 gene (genomic DNA) derived from the subject non-human mammal by a conventional method, and integrating a DNA strand having a DNA sequence constructed to inactivate the gene by, for example, (1) destroying the function of the exon or promoter by inserting another DNA fragment (for example, drug resistance gene, reporter gene and the like) into the exon portion or promoter region, (2) excising the entire or a portion of the GPR40 gene using the Cre-loxP system or Flp-frt system to delete the gene, (3) inserting a stop codon into the protein coding region to impede the translation into complete protein, or (4) inserting a DNA sequence that stops the transcription of the gene (for example, polyA addition signal and the like) into the transcription region to impede the synthesis of complete mRNA, (hereinafter, abbreviated as targeting vector), at the GPR40 gene locus of the subject non-human mammal by homologous recombination.

The homologous recombinant can be acquired by, for example, using the above-described targeting vector, in place of the DNA that encodes an exogenous GPR40, in the introduction of the DNA that encodes the exogenous GPR40 into an ES cell, a method of preparing the Tg animal of the present invention. If the targeting vector is one having a drug resistance or reporter gene inserted into the exon or promoter portion of the GPR40 gene, the transgenic ES cell can be selected with the drug resistance or reporter activity as the index. The drug resistance or reporter gene is preferably in the form of an expression cassette comprising an any promoter capable of functioning in a mammalian cell; however, if the reporter gene is inserted into the GPR40 gene so that the reporter gene is placed under the control of the endogenous promoter of the GPR40 gene, the vector for the reporter gene does not require a promoter.
Usually, gene recombination in a mammal occurs mostly non-homologously; the introduced DNA is randomly inserted at an any position on the chromosome. Therefore, it is not possible to efficiently select only those clones targeted to the endogenous GPR40 gene targeted by homologous recombination by selection based on the detection of the expression of a drug resistance or reporter gene and the like; it is necessary to confirm the site of integration by Southern hybridization or PCR for all the clones selected. Hence, provided that, for example, the herpes simplex virus-derived thymidine kinase (HSV-tk) gene, which confers gancyclovir sensitivity, is joined outside the region homologous to the target sequence of the targeting vector, the cells having the randomly inserted vector cannot grow in a gancyclovir-containing medium because they have the HSV-tk gene, whereas the cells having the endogenous GPR40 gene locus targeted by homologous recombination become resistant to gancyclovir and are selected because they do not have the HSV-tk gene. Alternatively, provided that the diphtheria toxin gene, for example, is joined in place of the HSV-tk gene, the cells having the randomly inserted vector die due to the toxin produced by themselves, so that a homologous recombinant can also be selected in the absence of a drug. The resulting resistant colonies are transferred to respective culture plates, and trypsinization and medium exchanges are repeated, after which a portion thereof is reserved for cultivation, whereas the remainder is subjected to PCR or Southern hybridization to confirm the presence of the introduced DNA.

Preparation of chimera embryos, establishment of founders, preparation of homozygotes and the like can be performed using the same methods as those described with respect to the method of preparing the Tg animal of the present invention using an ES cell.

Regarding specific means for knocking down the GPR40 gene, a method comprising introducing a DNA that encodes an antisense RNA or siRNA (including shRNA) of GPR40 using one of the above-described techniques of preparation of transgenic animals, and allowing it in the subject non-human mammal cell and the like can be mentioned.

A DNA comprising a base sequence complementary to the target region of a desired polynucleotide, i.e., a DNA hybridizable with a desired polynucleotide, can be said to be "antisense" against the desired polynucleotide.
The antisense DNA having a base sequence complementary or substantially complementary to the base sequence of a polynucleotide that encodes GPR40 or a portion thereof may be any antisense DNA, as long as it contains a base sequence complementary or substantially complementary to the base sequence of the polynucleotide that encodes GPR40 or a portion thereof, and having an action to suppress the expression of the polynucleotide.

The base sequence substantially complementary to a polynucleotide that encodes GPR40 is, for example, a base sequence having a homology of about 70% or more, preferably about 80% or more, more preferably about 90% or more, most preferably about 95% or more, to the base sequence of the complementary strand of the polynucleotide for the overlapping region. Base sequence homology herein can, for example, be calculated using the homology calculation algorithm NCBI BLAST (National Center for Biotechnology Information Basic Local Alignment Search Tool) under the following conditions (expect=10; gap allowed; filtering=ON; match score=1 mismatch score=-3).
Particularly, of the full base sequence of the complementary strand of the polynucleotide that encodes GPR40, (a) in the case of an antisense DNA intended to inhibit the translation, an antisense DNA having a homology of about 70% or more, preferably about 80% or more, more preferably about 90% or more, most preferably about 95% or more, to the complementary strand of the base sequence of the portion that encodes the N-terminal part of GPR40 protein (for example, a base sequence in the vicinity of the initiation codon and the like) is suitable, and (b) in the case of an antisense DNA intended to degrade RNA with RNaseH, an antisense DNA having a homology of about 70% or more, preferably about 80% or more, more preferably about 90% or more, most preferably about 95% or more, to the complementary strand of the full base sequence of the polynucleotide that encodes GPR40 including the intron, is suitable.

Specifically, when the subject non-human mammal is a mouse, an antisense DNA comprising a base sequence complementary or substantially complementary to the base sequence registered under GenBank accession No. NM_194057 (VERSION: NM_194057.1 GI:34610212) or a portion thereof, preferably, an antisense DNA comprising a base sequence complementary to the base sequence or a portion thereof, and the like can be mentioned.

An antisense DNA having a base sequence complementary or substantially complementary to the base sequence of a polynucleotide that encodes GPR40 or a portion thereof (hereinafter, also referred to as "the antisense DNA") can be designed and synthesized on the basis of base sequence information on a DNA that encodes cloned or determined GPR40. Such antisense DNA is capable of inhibiting the replication or expression of the GPR40 gene. Specifically, the antisense DNA is capable of hybridizing with an RNA transcribed from the GPR40 gene (mRNA or initial transcription product), and capable of inhibiting the synthesis (processing) or function (translation into protein) of mRNA.

The target region of the antisense DNA is not particularly limited with respect to the length thereof, as long as the translation into GPR40 protein is inhibited as a result of hybridization of the antisense DNA; the target region may be the entire sequence or a partial sequence of the mRNA that encodes the protein, and the length is about 10 bases for the shortest, and the entire sequence of the mRNA or initial transcription product for the longest. Specifically, the 5' end hairpin loop, 5' end 6-base-pair repeats, 5' end untranslated region, translation initiation codon, protein coding region, ORF translation stop codon, 3' end untranslated region, 3' end palindrome region, or 3' end hairpin loop of the GPR40 gene may be chosen as a preferable target region of the antisense DNA, but any other region in the GPR40 gene may also be chosen as the target. For example, the intron portion of the gene may also be the target region.
Furthermore, the antisense DNA may be one that not only hybridizes with the mRNA or initial transcription product of GPR40 to inhibit the translation into protein, but also is capable of binding to the GPR40 gene being a double-stranded DNA to form a triple strand (triplex) and hence to inhibit the transcription of RNA. Alternatively, the antisense DNA may be one that forms a DNA:RNA hybrid to induce the degradation by RNaseH.

A DNA that encodes a ribozyme capable of specifically cleaving the mRNA that encodes GPR40 or the initial transcription product within the coding region (including the intron portion in the case of the initial transcription product) can also be encompassed in the antisense DNA of the present invention. One of the most versatile ribozymes is a self-splicing RNA found in infectious RNAs such as viroid and virusoid, and the hammerhead type, the hairpin type and the like are known. The hammerhead type exhibits enzyme activity with about 40 bases in length, and it is possible to specifically cleave the target mRNA by making several bases at both ends flanking to the hammerhead structure portion (about 10 bases in total) a sequence complementary to the desired cleavage site of the mRNA. Because this type of ribozyme has only RNA as the substrate, it offers an additional advantage of non-attack of genomic DNA. Provided that the GPR40 mRNA assumes a double-stranded structure per se, the target sequence can be made to be single-stranded by using a hybrid ribozyme prepared by joining an RNA motif derived from a viral nucleic acid that can bind specifically to RNA helicase [Proc. Natl. Acad. Sci. USA, 98(10): 5572-5577 (2001)]. Furthermore, the ribozyme may be a hybrid ribozyme prepared by further joining a sequence modified from the tRNA to promote the translocation of the transcription product to cytoplasm [Nucleic Acids Res., 29(13): 2780-2788 (2001)].

Herein, a double-stranded DNA consisting of an oligo-RNA homologous to a partial sequence (including the intron portion in the case of the initial transcription product) in the coding region of the mRNA or initial transcription product of GPR40 and a strand complementary thereto, what is called a small interfering RNA (siRNA), can also be used to prepare the KD animal. It had been known that so-called RNA interference (RNAi), which is a phenomenon that when siRNA is introduced into cells, an mRNA homologous to the RNA is degraded, occurs in nematodes, insects, plants and the like; since this phenomenon was confirmed to also occur in animal cells [Nature, 411(6836): 494-498 (2001)], siRNA has been widely utilized as an alternative technique to ribozymes siRNA can be designed as appropriate on the basis of base sequence information of the mRNA being the target using commercially available software (e.g., RNAi Designer; Invitrogen).

The antisense oligo-DNA and ribozyme can be prepared by determining the target sequence for the mRNA or initial transcription product on the basis of a cDNA sequence or genomic DNA sequence of GPR40, and synthesizing a sequence complementary thereto using a commercially available DNA/RNA synthesizer (Applied Biosystems, Beckman, and the like). By inserting the synthesized antisense oligo-DNA or ribozyme downstream of the promoter in the expression vector, via an appropriate linker (adapter) sequence used as required, a DNA expression vector that encodes the antisense oligo-RNA or ribozyme can be prepared. Examples of expression vectors that can be used preferably here are as described above for the Tg animal of the present invention.
A DNA expression vector that encodes a longer antisense RNA (for example, full-length complementary strand of GPR40 mRNA and the like) can be prepared by inserting a GPR40 cDNA, cloned by a conventional method, in the reverse direction, via an appropriate linker (adapter) sequence used as required, downstream of the promoter in the expression vector.
Meanwhile, a DNA that encodes siRNA can be prepared by separately synthesizing a DNA that encodes a sense strand and a DNA that encodes an antisense strand, and inserting them into an appropriate expression vector. As the siRNA expression vector, one having a Pol III system promoter such as U6 or H1 can be used. In this case, in the animal cell incorporating the vector, the sense strand and the antisense strand are transcribed and annealed to form siRNA. shRNA can be prepared by inserting a unit comprising a sense strand and an antisense strand separated by a length allowing the formation of an appropriate loop structure (for example, about 15 to 25 bases) into an appropriate expression vector. As the shRNA expression vector, one having a Pol III system promoter such as U6 or H1 can be used. In this case, the shRNA transcribed in the animal cell incorporating the expression vector forms a loop by itself, and is then processed by an endogenous enzyme dicer and the like to form mature siRNA. Alternatively, it is also possible to achieve knockdown by RNAi by expressing a microRNA (miRNA) comprising the siRNA sequence being the target using a Pol II system promoter. In this case, by a promoter showing tissue-specific expression, tissue-specific knockdown is also possible.

For introducing an expression vector comprising a DNA that encodes an antisense RNA, siRNA, shRNA, or miRNA of GPR40 into a cell, any of the methods described above for the Tg animal is used as appropriate according to the target cell. For example, for introduction into an early embryo such as a fertilized egg, the microinjection method is used. For introduction into an ES cell, the calcium phosphate co-precipitation method, electroporation method, lipofection method, retrovirus infection method, aggregation method, microinjection method, particle gun method, DEAE-dextran method and the like can be used. Alternatively, when retrovirus, lentivirus and the like are used as the vector, it is sometimes possible to achieve gene introduction conveniently by adding the virus to an early embryo or an ES cell, and culturing the embryo or cell for 1 to 2 days to infect the cells with the virus.

Regeneration of individuals from an early embryo or ES cell (establishment of founder), passage (preparation of homozygotes) and the like can be performed as described above with respect to the Tg animal of the present invention.

In a congenic mouse obtained by back crossing the KO mouse of the present invention prepared on the basis of an ES cell derived from a 129/sv/Ev mouse to a C57BL/6J mouse, compared with wild type mice, no remarkable phenotypes were observed.

When the KO animal has been prepared by inserting a reporter gene into the GPR40 gene to destroy the gene, and the reporter gene is inserted at a position under the control of an endogenous promoter of the GPR40 gene, a drug regulating the promoter activity on the GPR40 gene can be screened for by applying a test substance to the animal or a portion of the living body thereof, and detecting the expression of the reporter gene. Examples of the reporter gene include, but are not limited to, DNAs that encode luciferase, peroxidase, green fluorescent protein (GFP), alkaline phosphatase, β-galactosidase and the like. Reporter activity can be measured by a method known per se according to the reporter gene used.
Useful test substances include, in addition to, commonly known synthetic compounds, peptides, proteins, DNA libraries and the like, for example, tissue extracts, cell culture supernatants and the like of mammals (for example, mice, rats, pigs, bovines, sheep, monkeys, humans and the like).
If a significant difference is observed in reporter activity, compared with an animal to which the test substance has not bee applied or a portion of the living body thereof, the test substance can be selected as a substance that regulates the promoter activity of the GPR40 gene.

Because the GPR40 promoter activity enhancing drug selected by the screening method is capable of having the same effect as the above-described GPR40 agonist, the same is useful as a prophylactic/therapeutic agent for diseases such as diabetes mellitus (type I and type II), impaired glucose tolerance, hyperlipemia, and metabolic syndrome, pancreas function regulator (e.g., pancreas function improving agent), insulin secretion promoter, hypoglycemic agent, and pancreatic β cell protecting agent. In addition, the GPR40 promoter activity enhancing drug can also be used as a prophylactic/therapeutic agent for diseases such as ketosis, acidosis, diabetic neuropathy, diabetic nephropathy, diabetic retinitis, arteriosclerosis, sexual dysfunction, dermatological disease, arthropathy, osteopenia, thrombotic disease, maldigestion, and memory and learning disturbance. The activity enhancing drug can be prepared as a pharmaceutical preparation in the same manner as with the GPR40 agonist, and can be administered orally or parenterally to mammals (for example, humans, rats, mice, guinea pigs, rabbits, sheep, pigs, bovines, horses, cats, dogs, monkeys and the like).

The dose of the GPR40 promoter activity enhancing drug varies depending on the target disease, subject of administration, route of administration and the like; for example, in the case of oral administration for treatment of diabetes mellitus, the usual dosage for an adult (weighing 60 kg) is about 0.1 mg to about 100 mg, preferably about 1.0 to about 50 mg, more preferably about 1.0 to about 20 mg, per day. In the case of parenteral administration, the dose of the regulatory drug varies depending on the subject of administration, target disease and the like; for example, in the case of administration as an injection to an adult (weighing 60 kg) for treatment of diabetes mellitus, the dose is about 0.01 to about 30 mg, preferably about 0.1 to about 20 mg, more preferably about 0.1 to about 10 mg, per day. If the subject of administration is a non-human animal, an amount converted per 60 kg of body weight can be administered.

Because the GPR40 promoter activity-suppressing drug selected by the screening method is capable of having the same effect as the above-described GPR40 antagonist, the same is useful as a prophylactic/therapeutic agent for diseases such as obesity, hyperlipemia, type II diabetes mellitus, and insulin resistance syndrome, pancreas function regulator (e.g., pancreas function improving agent), insulin secretion suppressant, and hyperglycemic agent. In addition, the GPR40 promoter activity-suppressing drug can also be used as a prophylactic/therapeutic agent for diseases such as hypoglycemia, hypertension, diabetic neuropathy, diabetic nephropathy, diabetic retinitis, edema, brittle diabetes mellitus, fat atrophy, insulin allergy, insulinoma, arteriosclerosis, thrombotic disease, fat toxicity, and cancers. The activity-suppressing drug can be prepared as a pharmaceutical preparation in the same manner as with the GPR40 agonist, and can be administered orally or parenterally to mammals (for example, humans, rats, mice, guinea pigs, rabbits, sheep, pigs, bovines, horses, cats, dogs, monkeys and the like).

The dose of the GPR40 promoter activity-suppressing drug varies depending on the target disease, subject of administration, route of administration and the like; for example, in the case of oral administration for treatment of obesity, the usual dosage for an adult (weighing 60 kg) is about 0.1 mg to about 100 mg, preferably about 1.0 to about 50 mg, more preferably about 1.0 to about 20 mg, per day. In the case of parenteral administration, the dose of the regulatory drug varies depending on the subject of administration, target disease and the like; for example, in the case of administration as an injection to an adult (weighing 60 kg) for treatment of obesity, the dose is about 0.01 to about 30 mg, preferably about 0.1 to about 20 mg, more preferably about 0.1 to about 10 mg, per day. If the subject of administration is a non-human animal, an amount converted per 60 kg of body weight can be administered.

The Tg animal of the present invention is not particularly limited with respect to the expression of endogenous GPR40, as long as an amount of exogenous GPR40 expressed is secured to the extent that enables a quantitative measuring of the action of the test substance on exogenous GPR40. However, for example, if the exogenous GPR40 is heterogeneous to the endogenous GPR40, and the Tg animal of the present invention is used to evaluate a drug capable of acting not only on the heterogeneous GPR40, but also on the endogenous GPR40, it is desirable that the expression of the endogenous GPR40 be inactivated. The Tg animal of the present invention having the expression of endogenous GPR40 inactivated can be obtained by introducing a DNA that encodes heterogeneous GPR40 into an ES cell having the GPR40 gene knocked out, selected by a commonly known method (see, for example, nonpatent document 3 above) or the method described above, or into an early embryo or ES cell prepared from the ES cell derived from the GPR40 KO animal by the above-described method, in accordance with the above-described method. Alternatively, the Tg animal of the present invention having the expression of endogenous GPR40 inactivated can also be obtained by, as described above, introducing a DNA that encodes heterogeneous GPR40 into an early embryo or ES cell derived from a KD animal having the expression of the GPR40 gene inactivated by antisense or RNAi technology, in accordance with the above-described method.

A Tg animal having the expression of endogenous GPR40 inactivated, and expressing heterogeneous GPR40 only, can also be obtained by mating an animal having the endogenous GPR40 knocked out (or knocked down) and an animal incorporating the above-described exogenous (heterogeneous) GPR40, of the same species as the KO (or KD) animal. For example, by further mating a male and a female of offspring obtained by mating an endogenous GPR40 homo-deficient (-/-) mouse and a human GPR40 Tg (+/-) mouse (endogenous GPR40 hetero-deficient (+/-)/human GPR40 hetero-Tg (+/-) mouse), and selecting a mouse confirmed to have human GPR40 Tg (+/-) and mouse GPR40 deficiency, an endogenous GPR40 (-/-) / human GPR40 Tg (+/-) mouse can be prepared.

Alternatively, a Tg animal having the expression of endogenous GPR40 inactivated may be a knockin (KI) animal having the endogenous GPR40 gene replaced with a DNA that encodes heterogeneous GPR40 by gene targeting using homologous recombination.

KI animals can be prepared using basically the same techniques as those for KO animals. For example, a targeting vector comprising a DNA obtained by excising a region comprising the ORF of the GPR40 gene derived from the subject non-human mammal using an appropriate restriction enzyme, and inserting the corresponding region of a heterogeneous GPR40 gene instead, may be introduced into an ES cell derived from the subject non-human mammal in accordance with the above-described method, and an ES cell clone having a DNA that encodes the heterogeneous GPR40 integrated at the animal's endogenous GPR40 gene locus by homologous recombination may be selected. The clone selection can be performed using PCR or Southern hybridization; for example, when a positive selection marker gene such as the neomycin resistance gene is inserted into the 3' untranslated region of the GPR40 gene of the targeting vector and the like, and a negative selection marker gene such as the HSV-tk gene or the diphtheria toxin gene is further inserted outside the region homologous to the target sequence, a homologous recombinant can be selected with drug resistance as an index.
Because a positive selection marker gene sometimes interferes with the expression of the introduced heterogeneous GPR40, it is preferable that the positive selection marker gene be cut out using a targeting vector wherein the loxP sequence or frt sequence is placed at both ends of a positive selection marker gene, by allowing a Cre or Flp recombinase or the recombinase expression vector (e.g., adenovirus vector and the like) to act at an appropriate time after homologous recombinant selection. Alternatively, in place of using the Cre-loxP system or the Flp-frt system, a sequence homologous to the target sequence may be arranged repeatedly in the same direction at both ends of a positive selection marker gene, and a positive selection marker gene may be cut out by means of intragenic recombination between the sequences.

Using an endogenous GPR40 KO/KD × heterogeneous GPR40 Tg animal or KI animal obtained as described above, as in the above-described exogenous GPR40 Tg animal, screening for GPR40 agonists and antagonists, or for insulin secretion/glucose tolerance regulatory drugs, can be performed. Because such KO/KD×Tg animals or KI animals do not express endogenous GPR40 or has the expression thereof reduced to a negligible level, the same are useful in that, for a drug that acts not only on heterogeneous GPR40, but also on endogenous GPR40, the action of the drug on heterogeneous GPR40 can be evaluated quantitatively.

In addition to stably retaining a DNA that encodes an exogenous GPR40 in an expressible state (Tg animal), and having the expression of an endogenous GPR40 gene inactivated (KO/KD animal), the Tg animal of the present invention and the KO/KD animal may have one or more other gene modifications that produce the same or similar condition as a disease in which GPR40 activity regulation is involved.
"A disease in which GPR40 activity regulation is involved" is to be understood as a concept encompassing not only diseases resulting from an abnormality in GPR40 activity or resulting in an abnormality in GPR40 activity, but also diseases on which a prophylactic and/or therapeutic effect can be obtained by regulating GPR40 activity.
For example, diseases that can be prevented/treated by activating GPR40 include diabetes mellitus (type I and type II), glucose tolerance impairment, hyperlipemia, and metabolic syndrome, as well as ketosis, acidosis, diabetic neuropathy, diabetic nephropathy, diabetic retinopathy, arteriosclerosis, sexual dysfunction, dermatological disease, arthropathy, osteopenia, thrombotic disease, maldigestion, memory and learning disturbance and the like, and diseases that can be prevented/treated by inhibiting GPR40 include obesity, hyperlipemia, type II diabetes mellitus, and insulin resistance syndrome, as well as hypoglycemia, hypertension, diabetic neuropathy, diabetic nephropathy, diabetic retinitis, edema, brittle diabetes mellitus, fat atrophy, insulin allergy, insulinoma, arteriosclerosis, thrombotic disease, fat toxicity, cancers and the like.

"Other gene modifications" include Tg animals spontaneous disease model animals having an abnormality in an endogenous gene thereof due to a spontaneous mutation, Tg animals further incorporating another gene, KO/KD animals having an endogenous gene other than the GPR40 gene inactivated (including Tg animals wherein gene expression has been reduced to an undetectable or negligible level by a gene destruction due to insertion mutation and the like, as well as introduction of an antisense DNA or a DNA that encodes a neutralizing antibody), dominant negative mutant Tg animals incorporating a mutant endogenous gene, and the like.

Examples of known "disease models having one or more other gene modifications that produce the same or similar condition as a disease in which GPR40 activity regulation is involved" include NOD mice (Makino S. et al., Exp. Anim., vol. 29, page 1, 1980), BB rats (Crisa L. et al., Diabetes Metab. Rev.), vol. 8, page 4, 1992), ob/ob mouse, db/db mouse (Hummel L. et al., Science, vol. 153, page 1127, 1966), KK mouse, KKA^{y} mouse, GK rat (Goto Y. et al., Tohoku J. Exp. Med., vol. 119, page 85, 1976), Zucker fatty rat (Zucker L.M. et al., Ann. NY Acad. Sci., vol. 131, page 447, 1965), ZDF rat, OLETF rat (Kawano K. et al., Diabetes, vol. 41, page 1422, 1992) and the like as diabetes mellitus models, ob/ob mice, db/db mice, KK mouse, KKA^{y} mouse, Zucker fatty rat, ZDF rat, OLETF rat and the like as obesity models, WHHL rabbits (having mutation in low density lipoprotein receptor (LDLR); Watanabe Y., Atherosclerosis, vol. 36, page 261, 1980), SHLM (spontaneous mice having apoE deficiency mutation; Matsushima Y. et al., Mamm. Genome, vol. 10, page 352, 1999), LDLR KO mouse (Ishibashi S. et al., J. Clin. Invest., vol. 92, page 883, 1993), apoE KO mouse (Piedrahita J.A. et al., Proc. Natl. Acad. Sci. USA, vol. 89, page 4471, 1992), human apo A/human apoB double Tg mouse (Callow M.J. et al., Proc. Natl. Acad. Sci. USA, vol. 91, page 2130, 1994) and the like as hyperlipemia or arteriosclerosis models, SPC2 KO mice (Furuta M. et al., Proc. Natl. Acad. Sci. USA, vol. 94, page 6646, 1997) and the like as hypoglycemia models, ob/ob mice (Herberg L. and Coleman D.L., Metabolism, vol. 26, page 59, 1977), KK mouse (Nakamura M. and Yamada K., Diabetologia, vol. 3, page 212, page 1967), FLS mouse (Soga M. et al., Lab. Anim. Sci., vol. 49, page 269, 1999) as a fatty liver model, CD55/CD59 double-Tg mice (Cowan P.J. et al., Xenotransplantation, vol. 5, pages 184-90, 1998) and the like as ischemic heart disease models, interleukin 1 Tg mice (Groves R.W. et al., Proc. Natl. Acad. Sci. USA, vol. 92, page 11874, 1995) and the like as dermatitis models, mice incorporating a mutant amyloid precursor protein gene and the like as Alzheimer's disease models, beta SAD (beta S-Antilles-D Punjab) Tg mice (Trudel M. et al., EMBO J, vol. 10, page 3157, 1991) and the like as anemic hypoxemia models, steroidogenic factor 1 KO mice (Zhao L. et al., Development, vol. 128, page 147, 2001) and the like as gonadal disorder models, p53 KO mice (Kemp C.J., Molecular Carcinogenesis, vol. 12, page 132, 1995) and the like as liver cancer models, c-neu Tg mice (Rao G.N. et al., Breast Cancer Res Treat, vol. 48, page 265, 1998) and the like as breast cancer models, and perforin/Fas-ligand double-KO mice (Spielman J. et al., J Immunol., vol. 161, page 7063, 1998) and the like as endometritis models.
These "disease models having other gene modifications" are purchasable from, for example, the Jackson Laboratory of the United States and the like, or can easily be prepared using a well known gene modification technology.

The method of introducing one or more other gene modifications that produce the same or similar condition as a disease in which GPR40 activity regulation is involved into the Tg animal of the present invention or the KO/KD animal is not particularly limited; examples include (1) a method comprising crossing the Tg animal of the present invention or the KO/KD animal and a non-human mammal of the same species having one or more other gene modifications that produce the same or similar condition as a disease in which GPR40 activity regulation is involved; (2) a method comprising introducing a DNA that encodes exogenous GPR40 into an early embryo or ES cell of a non-human mammal having one or more other gene modifications that produce the same or similar condition as a disease in which GPR40 activity regulation is involved, by the above-described method, to obtain a Tg animal (a method comprising treating an early embryo or ES cell of a non-human mammal having one or more other gene modifications that produce the same or similar condition as a disease in which GPR40 activity regulation is involved by the above-described method, to inactivate the expression of an endogenous GPR40 gene to obtain a KO/KD animal); (3) a method comprising introducing one or more other gene modifications that produce the same or similar pathologic condition as a disease in which GPR40 activity regulation is involved into an early embryo or ES cell of a non-human mammal incorporating DNA that encodes exogenous GPR40 by the above-described method (a method comprising introducing one or more other gene modifications that produce the same or similar pathologic condition as a disease in which GPR40 activity regulation is involved into an early embryo or ES cell of a non-human mammal having an endogenous GPR40 gene inactivated, by the above-described method) and the like. If one or more other gene modifications that produce the same or similar condition as a disease in which GPR40 activity regulation is involved are achieved by introducing an exogenous gene or dominant mutant gene, the exogenous gene and the like and a DNA that encodes exogenous GPR40 (or a targeting vector/ a DNA that encodes antisense RNA or siRNA) may be introduced simultaneously or sequentially into an early embryo or ES cell of a wild type non-human mammal to obtain a Tg (or KO/KD) animal. Furthermore, if one or more other gene modifications that produce the same or similar condition as a disease in which GPR40 activity regulation is involved are achieved by destroying an endogenous gene, a DNA that encodes exogenous GPR40 may be designed to be targeted to the endogenous gene to be destroyed, and introduced into an ES cell of a wild type non-human mammal. In this case, the targeting vector used is preferably the same as that exemplified with respect to the above-described preparation of a KI animal, except that the endogenous GPR40 gene is replaced with the endogenous gene to be destroyed.

If the Tg animal of the present invention or the KO/KD animal is crossed with a disease model non-human mammal of the same species having one or more other gene modifications that produce the same or similar condition as a disease in which GPR40 activity regulation is involved, it is desirable that homozygotes be crossed. For example, the F₁ obtained by crossing a homozygote mouse having a DNA that encodes exogenous GPR40 integrated at one gene locus and a db/db mouse (diabetes mellitus/obesity model) is heterolozygote with respect to both genes. Of the F₂ individuals obtained by mating siblings of this F₁, 1/16 are exogenous GPR40 (+/+)xdb/db.

The Tg animal of the present invention or the KO/KD animal may have undergone one or more non-genetic treatments that produce the same or similar condition as a disease in which GPR40 activity regulation is involved. "A non-genetic treatment" means a treatment that does not produce a gene modification in the subject non-human mammal. Examples of such treatments include, but are not limited to, induction with drugs such as STZ, dietary stress loads such as high-fat diet load, sugar load, and fasting, external stress loads such as UV, active oxygen, fever, and blood vessel ligation/reperfusion and the like.

The pathologic condition model animal obtained by subjecting the Tg animal of the present invention or the KO/KD animal to another gene modification or non-genetic treatment as described above can be used to screen for a substance having a prophylactic/therapeutic effect on a disease accompanied by the same or similar pathologic condition as the pathologic condition manifested by the animal. Specifically, by applying a test substance to the pathologic condition model animal or a portion of the living body thereof, and determining the amelioration of the pathologic condition, a prophylactic/therapeutic substance for a disease accompanied by the same or similar pathologic condition as the pathologic condition can be selected.
Specifically, preferable pathologic conditions include symptoms observed in metabolic syndrome, for example, diabetes mellitus, insulin resistance, glucose tolerance abnormality, obesity, hypertension, arteriosclerosis, hyper-TG-emia, hyper-LDL-C-emia, hypo-HDL-C-emia and the like.

Specifically, in the screening method, a test substance is administered to the above-described pathologic condition model animal. Useful test substances include, in addition to commonly known synthetic compounds, peptides, proteins, DNA libraries and the like, for example, tissue extracts, cell culture supernatants and the like of mammals (for example, mice, rats, pigs, bovines, sheep, monkeys, humans and the like).
If a significant amelioration of the pathologic condition is observed compared with animals not receiving the test substance, the test substance can be selected as a substance having a prophylactic/therapeutic activity for diseases accompanied by the pathologic condition.

The prophylactic/therapeutic drug for disease selected by the screening method can be prepared as a pharmaceutical preparation in the same manner as with the above-described GPR40 agonist, and can be administered orally or parenterally to mammals (for example, human, rats, mice, guinea pigs, rabbits, sheep, pigs, bovines, horses, cats, dogs, monkeys and the like).

The dose of the prophylactic/therapeutic drug varies depending on the target disease, subject of administration, route of administration and the like; for example, in the case of oral administration for treatment of diabetes mellitus, the usual dosage for an adult (weighing 60 kg) is about 0.1 mg to about 100 mg, preferably about 1.0 to about 50 mg, more preferably about 1.0 to about 20 mg, per day. In the case of parenteral administration, the dose of the prophylactic/therapeutic drug varies depending on the subject of administration, target disease and the like; for example, in the case of administration as an injection to an adult (weighing 60 kg) for treatment of diabetes mellitus, the dose is about 0.01 to about 30 mg, preferably about 0.1 to about 20 mg, more preferably about 0.1 to about 10 mg, per day. If the subject of administration is a non-human animal, an amount converted per 60 kg of body weight can be administered.

The sequence identification numbers in the sequence listing for the present invention show the following sequences:
[SEQ ID NO:1] Shows the base sequence of the DNA that encodes human GPR40 (CDS).
[SEQ ID NO:2] Shows the amino acid sequence of human GPR40.

In the present description, the abbreviations used to denote bases, amino acids and the like are based on abbreviations in accordance with the IUPAC-IUB Commission on Biochemical Nomenclature or on common abbreviations in the art. Some examples are given below.
DNA: Deoxyribonucleic acid
cDNA: Complementary deoxyribonucleic acid
A: Adenine
T: Thymine
G: Guanine
C: Cytosine
RNA: Ribonucleic acid
mRNA: Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediaminetetraacetic acid
SDS: Sodium dodecyl sulfate
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine
Gln: Glutamine
pGlu: Pyroglutamic acid
Me: Methyl group
Et: Ethyl group
Bu: Butyl group
Ph: Phenyl group
TC: Thiazolidine-4(R)-carboxamide group

### Examples

The present invention is hereinafter described in more detail by means of the following Examples, which, however, are not to be construed as limiting the scope of the invention.

### Example 1 Construction of expression vector for preparing human GPR40 gene transgenic mice

To highly express the human GPR40 gene in the mouse pancreas, the plasmid pISLII4/GPR40, incorporating the human GPR40 gene including the 3' untranslated region, downstream of the mouse insulin II promoter, was prepared as described below.
From a mouse genomic DNA (manufactured by CLONTECH), using a primer comprising HindIII and MluI restriction sites at the 5' end thereof (5'-ATTAGAAAGCTTACGCGTGAGAGATAGAGGAGGAGGGACCATTAAGTG-3'; SEQ ID NO:3), a primer comprising a SalI restriction site at the 5' end thereof (5'-GTCGACACAATAACCTGGAAGATAGGCTGGGTTGAGGATAGCAAA-3'; SEQ ID NO:4), and KOD polymerase (manufactured by Toyobo), PCR was performed under the conditions of 94°C 2 min → (94°C 15sec → 68°C 45sec) ×25 cycles, and a fragment comprising a mouse insulin II promoter was subcloned into pCR4-TOPO Blunt (manufactured by Invitrogen). Likewise, from a human genomic DNA (manufactured by CLONTECH), using a primer comprising an SalI restriction site at the 5' end thereof (5'-ATTATTGTCGACCACCATGGACCTGCCCCCGCAGCTCTCCTTCGGCCTCTATGTGG-3'; SEQ ID NO:5), a primer having the sequence 5'-TATGCACGCAAACACAAACTCTAT-3' (SEQ ID NO:6), and KOD polymerase (manufactured by Toyobo), PCR was performed under the conditions of 94°C 2 min → (94°C 15sec → 68°C 3min) ×25 cycles, a DNA fragment comprising a human GPR40 gene coding region and 3' untranslated region was subcloned into pCR4-TOPO Blunt (manufactured by Invitrogen).
The plasmid into which the mouse insulin II promoter had been subcloned was double digested with HindIII and SalI, the obtained 673-bp mouse insulin II promoter fragment was introduced at HindIII and SalI sites of pCAN618 (described in WO 00/14226). Next, a 2256-bp DNA fragment obtained by double digestion with SalI and SpeI from the plasmid into which the human GPR40 gene coding region and 3' untranslated region had been subcloned was inserted at the site resulting from double digestion of this plasmid with SalI and SpeI, to yield the human GPR40 gene expression plasmid pISLII4/GPR40 under the control of the mouse insulin II promoter. This was introduced into Escherichia coli DH5α to obtain the transformant Escherichia coli DH5α/pISLII4/GPR40. This strain has been deposited under accession number FERM BP-10462 at the International Patent Organism Depositary, National Institute of Advanced Industrial Science and Technology since December 1, 2005.
The above-described pISLII4/GPR40 was triple digested with MluI, KpnI, and SpeI, after which it was subjected to electrophoresis on low-melting-point agarose gel (manufactured by Takara Shuzo), and a 2928-bp DNA fragment was excised out. The DNA fragment was purified using Nucleotrap (manufactured by Japan Genetics), whereby a DNA fragment comprising a human GPR40 gene expression unit was acquired (FIG. 1A).

### Example 2 Preparation of human GPR40 gene transgenic mice

Preparation of transgenic mice by microinjection was performed in accordance with the method of Hogan et al. (Manipulating the mouse embryo, Cold Spring Harbor Laboratory Press, 1994). The DNA fragment comprising a human GPR40 gene expression unit, prepared in Example 1, was adjusted to obtain concentrations of 1 to 3.3 µg/ml in 1/10 TE solution (1 mM Tris-HCl, 0.1 mM EDTA, pH 8.0). A fertilized egg derived from the C57BL/6J strain was placed in a drop of M2 medium covered with mineral oil, and aspirated and immobilized using a holding pipette, and the above-described DNA solution was aspirated into an injection pipette, and injected into the male pronucleus of the fertilized egg using a micromanipulator (manufactured by Narishige). The fertilized egg receiving the injection was transplanted to a pseudopregnant female mouse, and the mouse was reared. Of the pups obtained, DNA were collected from the tails of 154 weaned mice when they reached 4 week-old. The acquired DNA were subjected to PCR using a primer set having the sequences 5'-GGAGTGTGGTGCTTAATCCGCTGGT-3' (SEQ ID NO:7) and 5'-AGACTGCCTCCTCCTTCCCGTAAGTACAA-3' (SEQ ID NO:8), and 27 transgenic mice were acquired.

### Example 3 Expression and genome analysis of human GPR40 gene transgenic mice

The acquired human GPR40 gene transgenic mice, at 8 week-old or after, were mated with C57BL/6J strain mice to obtain pups, from among which transgenic mouse individuals were selected by the same PCR method as Example 2. For each transgenic mouse pup, total RNA was extracted from the pancreas at 8 week-old using ISOGEN (manufactured by Nippon Gene) according to the attached protocol. One microgram of the obtained total RNA was treated with a random primer and SuperScript II reverse transcription enzyme (manufactured by Invitrogen) to synthesize a first strand cDNA; after ethanol precipitation, the precipitated cDNA was dissolved in 40 µl of TE. Of this solution, an aliquot of cDNA equivalent to 25 ng of RNA was used as the template for TaqMan analysis. Using a primer set of Forward primer (5'-GCCCGCTTCAGCCTCTCT-3'; SEQ ID NO:9), Reverse primer (5'-GAGGCAGCCCACGTAGCA-3'; SEQ ID NO:10), and FAM-labeled TaqMan primer probe (5'-TCTGCCCTTGGCCATCACAGCCT-3'; SEQ ID NO:11) for detection of the human GPR40 gene, TaqMan analysis (TaqMan 7700 and 7700SDS software, manufactured by Applied Biosystems) was performed. The working curve used to calculate copy number was generated from C_{T} values determined using known concentrations of a human GPR40 cDNA fragment comprising the amplified region in full length to obtain six logarithmic points from 10⁶ copies per well to 10¹ copies per well.
The expression of the human GPR40 gene in the pancreas in each strain of human GPR40 gene transgenic mice at 8 week-old is shown in FIG. 1B. In nearly all the strains examined, the expression of the human GPR40 gene was observed. The 47M strain and 81M strain exhibited higher expression levels than a plurality of strains including the 14M strain, 41M strain, and 23F strain.
For the above-described five strains of transgenic mice, i.e., the 14M strain, 41M strain, 47M strain, 81M strain, and 23F strain, genomic DNA was analyzed by Southern hybridization. Specifically, 5 µg of the DNA was cleaved with EcoRI and BglII, and 1.0% agarose gel electrophoresis was performed, after which the DNA was transferred to a nylon filter. This filter was hybridized with a probe of a DNA fragment containing the human GPR40 gene, previously labeled using a DIG RNA labeling kit (manufactured by Roche Diagnostics), overnight, washed twice with 2xSSC, 0.1% SDS at room temperature, and then washed twice with 0.1xSSC, 0.1% SDS at 68°C. For detection, a DIG fluorescence detection kit (manufactured by Roche Diagnostics) was used. As a result, an about 2.9-kbp band was identified from these five strains, confirming the introduction of the human GPR40 gene. It was also found that strains having the transgene integrated into the chromosome at high copy number were the 14M strain, 47M strain and 81M strain, and strains having the transgene integrated into the chromosome at low copy number were the 23F strain and 41M strain. There was a general correlation between the results for the amount of the human GPR40 gene expressed and the results for the copy number integrated into the chromosome; as a high expression strain, the 47M strain was chosen, as a standard expression system, the 23F strain was chosen, and the subsequent analysis was performed. The above-described mice were propagated and maintained by mating with mice of the C57BL/6J strain.

### Example 4 Expression of GPR40 gene in islets of Langerhans of human GPR40 gene transgenic mice

Starting at 8 week-old, transgenic mice of the 47M strain and 23F strain were fed with a low-fat diet (D12450B, 10 kcal% fat, manufactured by Research Diets, Inc.) or a high-fat diet (D12492, 60 kcal% fat, manufactured by Research Diets, Inc.) for 8 weeks. Each mouse at 16 week-old had the duodenal side ligated, and the common bile duct cannulated, and a 1 mg/ml collagenase solution (manufactured by Wako Pure Chemical) was injected into the mouse pancreas. The pancreas receiving the injection was excised, placed in a tube containing the same collagenase solution, and shaken at 37°C for 20 minutes. Subsequently, the solution was vigorously mixed for 30 seconds, and a Quenching buffer solution (Hanks Balanced Salt Solution comprising 0.001% DNaseI and 25 mM HEPES) containing 10% FBS was added to make a total volume of 20 ml. The solution was transferred to a Petri dish, and washed twice with the Quenching buffer solution, after which islets of Langerhans were recovered using a pipette under a microscopy, and this was used as the test sample. About 100 islets of Langerhans were isolated from each individual.
The isolated islets of Langerhans were homogenized in ISOGEN using an 18-gauge needle, and total RNA was extracted. Next, the extracted RNA was further purified using an RNeasy Micro kit (manufactured by QIAGEN), and finally DNase treatment was performed to remove contaminating DNA. Not more than 1 µg of total RNA was treated with a First strand cDNA synthesis kit (manufactured by Amersham Pharmacia Biotech) as directed in the protocol to synthesize a cDNA, which was used as the template for TaqMan analysis. For detection of the human GPR40 gene, TaqMan analysis was performed using the primer set described in Example 3. For detection of the mouse GPR40 gene, Assay on demand (manufactured by Applied Biosystems) was used; for detection of the 18S ribosome RNA gene being the internal standard, the Taqman Ribosomal RNA Control Reagents VIC Probe set (manufactured by Applied Biosystems) was used. The working curve used to calculate copy numbers was generated from C_{T} values determined using known concentrations of a human GPR40 cDNA, a mouse GPR40 cDNA, or a DNA fragment that encodes mouse 18S ribosome RNA gene, comprising the amplified region in full length, to obtain four logarithmic points from 10⁶ copies per well to 10³ copies per well.
As shown in FIG. 2A, the human GPR40 gene introduced was expressed in the isolated islets of Langerhans only in the transgenic mice of 47M strain and 23F strain, and the amount expressed was higher in 47M, and correlated with the expression level in the pancreas (see FIG. 1B). For both the 47M strain and 23F strain, there was no significant difference in the expression level of human GPR40 gene between the low-fat diet load group and the high-fat diet load group. Meanwhile, for both the 47M strain and 23F strain, in terms of the amount of mouse endogenous GPR40 gene expressed, there was no significant difference between the control mice and the transgenic mice; the expression level of mouse GPR40 gene was nearly at the same level between the low-fat diet load group and the high-fat diet load group (FIG. 2B). The expression level of human GPR40 gene in islets of Langerhans increased 70 times in the 47M strain, and about 30 times in the 23F strain, compared with that of endogenous GPR40 gene in mouse; the transgenic mice prepared were expected to have a physiologically sufficient action.

### Example 5 General properties and glucose tolerance test of human GPR40 gene transgenic mice

Transgenic mice of the 47M strain and 23F strain were fed with a normal diet (CE-2, 12 kcal% fat, manufactured by Clea Japan). After each mouse was weighed, blood was drawn from the ocular fundus using a heparinized capillary (manufactured by Drummond Scientific Company), and plasma was acquired via centrifugation. Regarding plasma components, glucose levels were determined using Fuji Drychem (manufactured by Fuji Film Medical), and insulin levels were determined using a Morinaga insulin ELISA kit (manufactured by Biochemical Research Laboratory, Morinaga Milk Industry Co., Ltd.).
A glucose tolerance test was performed as described below. Specifically, 16 hours after the start of fasting, blood was drawn from the ocular fundus of each mouse using a heparinized capillary, and this was used as the 0-minute sample. Next, a 10% glucose solution was administered orally at 1 g per kg of body weight; 7.5 minutes, 15 minutes, 30 minutes, 60 minutes, and 120 minutes after the administration, blood was drawn from the ocular fundus using a heparinized capillary, and these were used as the samples for the respective time points. These samples were centrifuged to isolate plasma components, and glucose and insulin levels were measured as described above.
At 16 week-old, for both the 47M strain and 23F strain, in terms of body weight values at fed and fasting, there was no difference among the respective control mice (Table 1). Fasting plasma glucose levels tended to be lower in the transgenic mice (Table 1). A glucose tolerance test was performed on mice of the 47M strain at 16 week-old and mice of the 23F strain at 18 week-old; the plasma glucose level was lower in the transgenic mice of 47M strain and 23F strain than in the control mice, whereas the plasma insulin level was higher in the transgenic mice (FIG. 3). These results are thought to show that in the transgenic mice, the glucose tolerance improved with enhancement of insulin secretion. Since similar results were obtained from the two strains of transgenic mice, the 47M strain was mainly used for the analysis that followed.

**[Table 1]**

| Properties of Transgenic Mice | | | | | |
|---|---|---|---|---|---|
| **A** | body weight (g) | | plasma glucose(mg/dl) | plasma insulin (ng/ml) | |
| | satiety control | 28.8±0.4 | 170.0±4.1 | 1.54±0.30 | 16 week-old n=7-8 |
| | Tg:47M | 27.5±0.6 | 153.6±6.2 | 1.61±0.43 | |
| | fasting control | 24.3±0.4 | 106.0±4.8 | 0.19±0.04 | |
| | Tg:47M | 22.9±0.5 | 84.3±4.4 | 0.23±0.05 | |
| | | | | | |

| **B** | body weight (g) | | plasma glucose(mg/dl) | plasma insulin(ng/ml) | |
|---|---|---|---|---|---|
| | satiety control | 28.7±0.4 | 187.6±9.8 | 1.43±0.38 | 16 week-old n=7-8 |
| | Tg:23F | 28.2±0.3 | 162.4±7.1 | 1.51±0.34 | |
| | fasting control | 24.0±0.3 | 117.7±5.0 | 0.21±0.03 | |
| | Tg:23F | 23.8±0.3 | 89.9±4.5 | 0.26±0.05 | |
| | | | | mean±SE | |

### Example 6 Glucose tolerance test of human GPR40 gene transgenic mice under fatty diet loads

To examine the effects under high-fat diet conditions, transgenic mice of the 47M strain mice were fed with a low-fat diet (D12450, 10 kcal% fat, manufactured by Research Diets, Inc.), a high-fat diet containing 45 kcal% fat (D12451, manufactured by Research Diets, Inc.), and a high-fat diet containing 60 kcal% fat (D12492, manufactured by Research Diets, Inc.) for 9 weeks from 8 week-old, after which a glucose tolerance test was performed on the mice at 17 week-old.
As shown in FIG. 4, the glucose tolerance of control mice worsened in the 45 kcal% fat high-fat diet load group and the 60 kcal% fat high-fat diet load group compared with the low-fat diet load group; effects of the high-fat diet were evident. Comparing the control mice and the transgenic mice of the 47M strain, the glucose tolerance of transgenic mice was better than that of the control mice, in all the low-fat diet load group, 45 kcal% fat high-fat diet load group, and 60 kcal% fat high-fat diet load group. In all cases, insulin secretion tended to increase in the transgenic mice, and this tendency was remarkable in the high-fat diet load group. At the same time, in insulin sensitivity, there was no major difference between the control mice and the transgenic mice. These results are thought to show that in the transgenic mice, the glucose tolerance improved with enhancement of insulin secretion. In the high-fat diet load groups, enhancement of insulin secretion was remarkable; it was suggested that these results were based on the GPR40 gene.

### Example 7 Insulin secretion from isolated islets of Langerhans derived from human GPR40 gene transgenic mice upon glucose stimulation

Insulin secretion from isolated islets of Langerhans derived from transgenic mice of the 47M strain in response to glucose stimulation was examined. Islets of Langerhans were isolated from mice fed with a normal diet at 10 to 13 week-old by the method described in Example 4. After cultivation in an RPMI1640 medium (manufactured by Invitrogen) containing 11 mM glucose, 1 mM HEPES, and 10% FBS for 16 hours, the islets of Langerhans were cultured in a Krebs Ringer bicarbonate buffer (116 mM NaCl, 4.7 mM KCl, 1.17 mM KH₂PO₄, 1.17 mM MgSO₄-7H₂O, 25 mM NaHCO₃, 2.52 mM CaCl₂, 24 mM HEPES, 0.2% BSA) containing 1 mM glucose and 0.2% BSA for 30 minutes. Next, the medium was replaced with a Krebs Ringer bicarbonate buffer containing 3 mM glucose or 16 mM glucose, and the islets of Langerhans were cultured for 1 hour. In the case of an experiment with the addition of palmitic acid, palmitic acid was added to a Krebs Ringer bicarbonate buffer containing 11 mM glucose to obtain a concentration of 0.5 mM, and islets of Langerhans were cultured for 1 hour. After cultivation, the culture supernatant was assayed for insulin content using a Morinaga insulin ELISA kit (manufactured by Biochemical Research Laboratory, Morinaga Milk Industry Co., Ltd.). Meanwhile, after cultivation, islets of Langerhans were disrupted by sonication using a sonicator (manufactured by M & S Instruments Trading Inc.), the amount of DNA was determined using a Quant-iT^{™} Picogreen ds DNA Assay kit (manufactured by Molecular Probes), and the amount of insulin secreted was corrected.
In the amount of insulin secreted in the presence of 3 mM glucose, no difference was observed between the control mice and the 47M strain (FIG. 5A). In contrast, in the presence of 16 mM glucose, the amount of insulin secreted was higher in the 47M strain than in the control mice (FIG. 5A). In the experiment with the addition of palmitic acid, one of GPR40 ligand, the amount of insulin secreted increased about 1.5 times in the palmitic acid added group of control mice, compared with the no-addition group, and the amount of insulin increased about 6 times in the transgenic mice, compared with the no-addition group (FIG. 5B). From these results, it was found that the amount of insulin secreted upon stimulation with high concentration glucose in islets of Langerhans derived from the transgenic mice was higher than that of the control mice, and that its responsivility to palmitic acid was higher than that of the control mice. It is suggested that both may be attributed to the high expression of the GPR40 gene.

### Example 8 Acquisition of homologous recombinant ES cells for preparation of GPR40 gene deficient mice

The GPR40 gene targeting vector is as described in Example 21 of patent document 3. Escherichia coli transformed with the vector, Escherichia coli DH5α/pGT-GPR40, has been deposited under accession number FERM BP-8259 at the International Patent Organism Depositary, National Institute of Advanced Industrial Science and Technology since December 11, 2002.
Twenty micrograms of the GPR40 gene targeting vector, linearized with restriction enzyme NotI, was introduced into ES cells (AB2.2 prime, manufactured by Lexicon) by electroporation. From among the selected 384 clones of G418-resistant strains, homologous recombinant candidate strains were selected by PCR. Specifically, using Primer #1 and #2 (#1: 5'-CAGCCAGTCCCTTCCCGCTTCA-3' (SEQ ID NO:12), a sequence within the Neo^{r} in the targeting vector, #2: 5'-GCAGGTCCGAAATGGTCAGGTTTAGCA-3' (SEQ ID NO:13), a sequence outside the 3' arm of the targeting vector) and LA Taq polymerase (manufactured by Takara Shuzo), PCR was performed at 94°C for 1 min → (98°C 10sec → 70°C 5 min 30sec) ×35 cycles → 72°C 10 min. As a result, from 7 positive clones and 12 false-positive clones, i.e., a total of 19 clones, an about 5.1-kbp band was obtained. Out of them, 10 clones including the 7 positive clones, were again examined by PCR under the same conditions; seven clones, i.e., Nos. 85, 231, 247, 325, 373, 374, and 391, were positive.
Next, to examine for the presence or absence of homologous recombination at the GPR40 gene locus, Southern hybridization was performed. Specifically, with a 1.2-kbp SacI-EcoRI fragment being present outside the 3' arm of the targeting vector as the probe, the DNA of the ES cell was digested with EcoRI, after which Southern hybridization was performed. Thereby, a 9.7-Kbp band is obtained from the wild allele, and from the targeted allele a 4.6-kbp band is obtained. With a 0.9-kbp SacI-BamHI fragment being present outside the 5' arm of the targeting vector as the probe, the DNA of the ES cell was digested with SacI, after which Southern hybridization was performed. Thereby, a 14.2-Kbp band is obtained from the wild allele, and a 17.4-kbp band is obtained from the targeted allele.
On 9 clones, including the 7 clones judged to be positive in the foregoing PCR, Southern hybridization was performed. For the 3' side arm, Southern hybridization was performed as described below. After 2 µg of DNA of each clone and mouse genomic DNA were digested with EcoRI, electrophoresis was performed on 0.4% TAE agarose gel, and the DNA was blotted to a nylon filter. The EcoRI-SacI 1.2-kbp DNA fragment outside the 3' side arm was labeled using [α-³²P] dCTP (NEG513Z, manufactured by DuPont) by a random prime method (Multiprime DNA labeling system RPN.1601Y, manufactured by Amersham Pharmacia Biotech), and this was used as the probe. Hybridization was performed in a hybridization buffer (0.5M Na⁺- phosphate buffer pH 7.2, 7% SDS, 1% BSA, 1mM EDTA) at 65°C overnight. Finally, washing with 0.1×SSC, 0.1% SDS was performed for 20 minutes x 2, and an autoradiogram was taken using BAS2000 (manufactured by Fuji Films). For the 5' side arm, 2 µg of DNA of each clone and mouse genomic DNA were digested with SacI, and with the SacI-BamHI 0.9-kbp DNA fragment outside the 5' side arm as the probe, Southern hybridization was performed as described above. As a result, both the 4.6-kbp band observed when homologous recombination has occurred in the 3' side arm region, and the 17.4-kbp band observed when homologous recombination has occurred in the 5' side arm region, were obtained from six clones, i.e., Nos. 85, 231, 247, 325, 373, and, 374; these six clones became homologous recombinant candidate strains. However, since the 17.4-kbp band was heterologous in some clones, this region was further analyzed.
To confirm that the targeting vector was not inserted into a position other than the homologous recombination region on the chromosome, or not tandemly inserted into the homologous recombination site, and the like, the membrane filter used in the Southern hybridization analysis of the region outside the 5' side arm was reprobed, after which Southern hybridization was performed with the 1.9-kbp BamHI-EcoRI fragment corresponding to the Neo^{r} region in the targeting vector as the probe. If accurately one copy of the homologous recombinant is inserted, the 17.4-kbp band will be detected. In No. 247, 373, and 374 clones, a plurality of bands were observed; therefore, the targeting vector was likely to be inserted on the genome by non-homologous recombination in these clones. Regarding No. 85 clone, only one band was obtained, but because the band was denser than that of any other clone, it was suggested that the targeting vector might be tandemly integrated into the homologous recombination region in this clone. Finally, two clones from which only single 17.4-kbp band was obtained, i.e., Nos. 231 and 325, were identified as homologous recombinants.

### Example 9 Preparation of GPR40 gene homo-deficient mice and expression of GPR40 gene in homo-deficient mice

The homologous recombinant ES cell strain No. 231 was injected into a blastocyst derived from a mouse of the C57BL/6J strain. The injected blastocyst was transplanted to the oviduct of a pseudopregnant mouse, and 31 chimera mice were obtained. Male mice showing a high chimera ratio with an ES cell contribution rate of 50% or more were mated to obtain 106 ES-cell-derived mice, whereby germline transmission was confirmed. Genomic DNA was purified from the tails of these mice, and genotyping was performed by PCR using the primer #1 and #2 described in Example 8; it was found that 48 individuals were hetero-deficient mice. Next, genotyping of the pups obtained by mating the hetero-deficient mice was performed as described below. With DNA prepared from the tail as the template, using the primer #1 and primer #2 described in Example 8, PCR was performed under the conditions of 94°C 1 min → (98°C 10sec → 70°C 5min 30sec) ×25 cycles → 72°C 10 min. No bands are detected in samples from wild mice, whereas an about 5.1-kbp band is detected in samples from hetero-deficient mice and homo-deficient mice. Likewise, using primer #3 (5'-GCCCGCCCTGCCCGTCTCA-3' (SEQ ID NO:14), a sequence in the lacked portion of the mouse GPR40 gene) and primer #4 (5'-AACGTTCGATGCTCACCGCCGTCA-3' (SEQ ID NO:15), a sequence outside the 3' side arm of the targeting vector), PCR was performed under the conditions of 94°C 1 min → (98°C 10sec → 70°C 5min 30sec) x30 cycles → 72°C 10 min. No bands are detected in samples from homo-deficient mice, whereas an about 5.4-kbp band is detected in samples from wild mice and hetero-deficient mice. The wild mice, hetero-deficient mice, and homo-deficient mice were acquired as described above. Thereafter, these mice were maintained and propagated by mating homo-deficient mice, and the control mice were maintained and propagated by mating wild mice; these mice were supplied for the experiments.
For the GPR40 gene homo-deficient mice, GPR40 gene hetero-deficient mice, and control mice, total RNA was extracted from the pancreas at 8 week-old using ISOGEN, as directed in the attached protocol. One microgram of the total RNA obtained was treated with random primers and SuperScript II reverse transcription enzyme as directed in the attached protocol to synthesize a first strand cDNA; after ethanol precipitation, the precipitated cDNA was dissolved in 40µl of TE. Of this solution, an aliquot of cDNA equivalent to 25 ng of RNA was used as the template for TaqMan analysis. For detection of the mouse GPR40 gene, a primer set of forward primer (5'-TTTGCGCTGGGCTTTCC-3'; SEQ ID NO:16), reverse primer (5'-GCTGGGAGTGAGTCGCAGTT -3'; SEQ ID NO:17), and a FAM-labeled TaqMan primer probe (5'-CCATCCGAGGCGCAGTGTCCC-3'; SEQ ID NO:18) was used; for detection of the actin gene, a primer set of forward primer (5'-CGTGAAAAGATGACCCAGATCA-3'; SEQ ID NO:19), reverse primer (5'-CACAGCCTGGATGGCTACGT-3'; SEQ ID NO:20), and a FAM-labled TaqMan primer probe (5'-TGAGACCTTCAACACCCCAGCCATG-3'; SEQ ID NO:21) was used. The working curve used to calculate copy numbers was generated from C_{T} values determined using known concentrations of a mouse GPR40 gene containing a plasmid DNA comprising the amplified region in full length, or a synthetic DNA fragment being a portion of the mouse actin gene (manufactured by SIGMA Genosys, 5'-CCAACCGTGAAAAGATGACCCAGATCATGTTTGAGACCTTCAACACCCCAGCCATGTACGTAG CCATCCAGGCTGTGCTGTC-3'; SEQ ID NO:22), to obtain six logarithmic points from 10⁶ copies per well to 10¹ copies per well. The expression level of the mouse GPR40 gene is expressed as a ratio to the expression level of the actin gene.
As shown in FIG. 6, the mouse GPR40 gene expression level in the pancreases of the control mice was evident, whereas the expression level in the pancreases of the GPR40 gene hetero-deficient mice had decreased to about half as high as that of the control mice, and the expression in the pancreases of the GPR40 gene homo-deficient mice had disappeared. From these results, it was found that the expression level in these mice correlated with the amount of the mouse GPR40 gene, and it was expected that the GPR40 gene homo-deficient mice acquired would exhibit phenotypes associated with the GPR40 gene deficiency.
Next, by mating the above-described hetero-deficient mouse or homo-deficient mouse with a C57BL/6J mouse, hetero-deficient mice were acquired (first generation). The hetero-deficient mice obtained by repeating this mating five times (fifth generation) were mated to obtain control mice and homo-deficient mice. Thereafter, these mice were maintained and propagated by mating hetero-deficient mice, or control mice or homo-deficient mice; these mice were supplied for the experiments.

### Example 10 General properties of GPR40 gene homo-deficient mice

Starting at 8 week-old, the GPR40 gene homo-deficient mice were fed with a low-fat diet (D12450B, 10 kcal% fat, manufactured by Research Diets, Inc.) or a high-fat diet (D12492, 60 kcal% fat, manufactured by Research Diets, Inc.) for 8 weeks. Starting after the initiation of fatty diet loading, each mouse was weighed weekly, with blood parameters were measured as necessary. Specifically, blood was drawn from the ocular fundus using a heparinized capillary, plasma components were acquired via centrifugation, and plasma glucose levels and plasma insulin levels were measured by the method described in Example 5. Daily calorific intake was measured as described below. On the day before the start of measurement, the weight of the food on the feeding wire net was measured, and on the following day the weight of the food on the wire net and the weight of the food scattered in the cage were measured, whereby the weight of the food taken was determined, and this was multiplied by the calorific value of each food to calculate daily calorific intake.
For both the low-fat diet load group and the high-fat diet load group, the body weight value of the GPR40 gene homo-deficient mice was equal to that of the control mice (FIG. 7A and E), and in terms of calorific intake, there was no difference between the homo-deficient mice and the control mice (FIG. 7B and F). For both the low-fat diet load group and the high-fat diet load group, in terms of both plasma glucose levels and plasma insulin levels, no significant difference was observed between the control mice and the homo-deficient mice (FIG. 7C, D, G and H). Hence, it was thought that in terms of body weight, calorific intake, plasma glucose level, and plasma insulin level, there was no major difference between the GPR40 gene homo-deficient mice and the control mice.

### Example 11 Glucose tolerance of GPR40 gene homo-deficient mice

Starting at 8 week-old, the GPR40 gene homo-deficient mice were fed with a low-fat diet (D12450B, 10 kcal% fat, manufactured by Research Diets, Inc.) or a high-fat diet (D12492, 60 kcal% fat, manufactured by Research Diets, Inc.) for 11 weeks. On mice at 19 week-old, a glucose tolerance test was performed in the same manner as Example 5.
For both the low-fat diet group and the high-fat diet group, there was no major difference in glucose tolerance between the control mice and the homo-deficient mice. From these results, it was thought that the deficiency of the GPR40 gene did not influence the glucose tolerance.

### Example 12 Hybrid mice of human GPR40 gene transgenic mice and GPR40 gene homo-deficient mice

To prepare mice that express the human GPR40 gene only, the following was performed. Specifically, by crossing a human GPR40 gene transgenic mouse of the 47M strain acquired in Example 3 and a GPR40 gene homo-deficient mouse acquired in Example 9, individuals retaining the human GPR40 gene, and having heterozygous for deficiency of the mouse GPR40 gene, were acquired. The male transgenic mice thus obtained were crossed with female GPR40 gene homo-deficient mice, and the female transgenic mice were crossed with male GPR40 gene homo-deficient mice, whereby hybrid mice retaining the human GPR40 gene, and having homozygous for deficiency of the mouse GPR40 gene (47M TgxKO), were acquired. At the same time, control mice having homozygous for deficiency of the mouse GPR40 gene, and not having the human GPR40 gene (NonTgxKO), were also acquired. Subsequently, mice obtained by crossing 47M TgxKO and NonTgxKO were examined for the presence or absence of the human GPR40 gene, whereby 47M TgxKO and NonTgxKO were selected. For the human GPR40 gene transgenic mice of the 23F strain as well, hybrid mice retaining the human GPR40 gene, and having homozygous for deficiency of mouse GPR40 gene (23F TgxKO) and control mice therefor (NonTgxKO) were prepared as described above. The 47M TgxKO and 23F TgxKO mice are thought to be mice characterized by the loss of the expression of the mouse GPR40 gene and the expression of the human GPR40 gene only.

### Example 13 Human GPR40 gene transgenic mice having the genetic background of KKA^{y} mice

To examine the effects of high expression of GPR40 gene on KKA^{y} mice, which exhibit hyperglycemia and obesity, human GPR40 gene transgenic mice having the genetic background of KKA^{y} mice were prepared. Specifically, by crossing human GPR40 gene transgenic mice of the 47M strain acquired in Example 3 and KKA^{y} mice, mice retaining the human GPR40 gene, and having the A^{y} gene as determined from the coat color (47M Tg/KKA^{y}), were acquired. At the same time, mice retaining the human GPR40 gene, but not having the A^{y} gene (47M Tg/KK), were also acquired. Likewise, 23F Tg/KKA^{y} and 23F Tg/KK were also prepared. In either case, NonTg/KKA^{y} and NonTg/KK served as control mice, respectively.

### Industrial Applicability

Because the Tg and KO/KD animals exhibit phenotypes that are more reflective of the normal GPR40 functions, they are useful as screening and drug efficacy evaluation systems in vivo for GPR40 action regulatory drugs.

While the present invention has been described with emphasis on preferred embodiments, it is obvious to those skilled in the art that the preferred embodiments can be modified. The present invention intends that the present invention can be embodied by methods other than those described in detail in the present specification. Accordingly, the present invention encompasses all modifications encompassed within the scope of the appended claims.

## Claims

1. A transgenic mouse in which the insulin secretion capacity has been increased and/or the glucose tolerance has been improved as compared with the corresponding non-transgenic mouse, **characterized in that** the mouse expresses an exogenous GPR40 gene under the control of an insulin II promoter, or a body-part thereof.

2. The mouse according to claim 1, wherein the exogenous GPR40 has the same or substantially the same amino acid sequence as the amino acid sequence shown by SEQ ID NO:2, or a body-part thereof.

3. A screening method for an GPR40 agonist or a GPR40 antagonist, comprising applying a test compound to the mouse according to claim 1, or a body-part thereof, and determining the GPR40 agonist activity or GPR40 antagonist activity.

4. A screening method for an (1) insulin secretion and/or (2) glucose tolerance regulatory drug, comprising applying a test compound to the mouse according to claim 1, or a body-part thereof, and measuring the (1) insulin secretion and/or (2) glucose tolerance.

5. The mouse according to claim 1, wherein the exogenous GPR40 is heterogeneous to the mouse, and the mouse is deficient in the expression of the endogenous GPR40 gene, or a body-part thereof.

6. The mouse according to claim 5, wherein the heterogeneous GPR40 is derived from human, or a body-part thereof.

7. The screening method according to claim 3, which is a screening method for a heterogeneous GPR40 agonist or a heterogeneous GPR40 antagonist, comprising applying a test compound to the mouse according to claim 5, or a body-part thereof, and determining the GPR40 agonist activity or GPR40 antagonist activity.

8. The screening method according to claim 4, which is a screening method for an (1) insulin secretion and/or (2) glucose tolerance regulatory drug in a heterogeneous mammal, comprising applying a test compound to the mouse according to claim 5, or a body-part thereof, and measuring the (1) insulin secretion and/or (2) glucose tolerance.

9. A mouse expressing an exogenous GPR40 gene under the control of an insulin II promoter and having one or more gene modification(s) other than GPR40 gene that produce the same or similar condition as a disease in whch GPR40 activity regulation is involved, or a body-part thereof, wherein said mouse results from mating of a mouse expressing an exogenous GPR40 gene which is homozygous under the control of an insulin II promoter and a disease model mouse having one or more gene modifications other than GPR40 gene that produce the same or similar condition as a disease in which GPR40 activity regulation is involved.

10. A screening method for a prophylactic/therapeutic substance for a disease in which GPR40 activity regulation is involved , comprising applying a test compound to the mouse according to claim 9, or a body-part thereof, and determining the amelioration of the disease.

11. The method according to claim 10, wherein the disease is metabolic syndrome or one or more symptoms thereof.

## Patentansprüche

1. Transgene Maus, in welcher die Insulin Sekretionskapazität erhöht worden ist und/ oder die Glukose Toleranz im Vergleich mit der entsprechenden nichttransgenen Maus verbessert worden ist, **gekennzeichnet dadurch, daß** die Maus oder ein Körperteil davon ein exogenes GPR40 Gen unter Kontrolle eines Insulin II Promoters exprimiert.

2. Maus oder Körperteil davon nach Anspruch 1, wobei exogenes GPR40 dieselbe oder im wesentlichen dieselbe Aminosäuresequenz wie die Aminosäuresequenz, gezeigt durch SEQ ID Nr: 2, hat.

3. Screening-Verfahren für einen GPR40 Agonisten oder einen GPR40 Antagonisten, umfassend Anwenden einer Test-Verbindung an der Maus oder Körperteils davon nach Anspruch 1 und Bestimmen der GPR40 Agonist Aktivität oder GPR40 Antagonist Aktivität.

4. Screening-Verfahren für (1) Insulin Sekretion und/ oder (2) Glukose-Toteranz regulierender Wirkstoff, umfassend Anwenden einer Test-Verbindung an der Maus oder Körperteils davon, nach Anspruch 1 und Messen der (1) Insulin Sekretion und/ oder (2) Glukose-Toleranz.

5. Maus oder Körperteil davon nach Anspruch 1, wobei das exogene GPR40 heterogen zur Maus ist und die Maus endogenes GPR40 mangelhaft exprimiert.

6. Maus oder Körperteil davon nach Anspruch 5, wobei heterogenes GPR40 vom Menschen abstammt.

7. Screening Verfahren nach Anspruch 3, welches ein Sreening Verfahren für einen heterogenen GPR40 Agonisten oder einen heterogenen GPR40 Antagonisten ist, umfassend Anwenden einer Test-Verbindung an der Maus oder Körperteils davon nach Anspruch 5 und Bestimmen der GPR40 Agonisten Aktivität oder GPR40 Antagonisten Aktivität.

8. Screening Verfahren nach Anspruch 4, welches ein Screening Verfahren für (1) Insulin Sekretion und/ oder (2) Glukose Toleranz regulatorischen Wirkstoff in einem heterogenen Säugetier ist, umfassend Anwenden einer Test-Verbindung an der Maus oder Körperteils davon nach Anspruch 5 und Messen der (1) Insulin Sekretion und/ oder (2) Glukose Toleranz.

9. Maus oder Körperteil davon exprimierend exogenes GPR40 Gen unter der Kontrolle eines Insulin II Promoters und ein oder mehrere andere Gen-Modifikation(en) als das GPR40 Gen habend, das diesselbe oder ähnliche Bedingung als Krankheit produziert, in welcher GPR40 Aktivitätsregulation beteiligt ist, wobei die Maus aus Verpaaren einer Maus, die ein exogenes GPR40 Gen exprimiert, welches homozygot unter der Kontrolle eines Insulin II Promoters und eines Krankheits-Maus Modells resultiert, die ein oder mehrere andere Gen-Modifikationen als das GPR40 Gen hat, das diesselbe oder ähnliche Bedingung als eine Krankheit produziert, in welcher GPR40 Aktivitätsregulation beteiligt ist.

10. Screening Verfahren für eine prophylaktische/ therapeutische Substanz für eine Krankheit, in welcher GPR40 Aktivitätsregulation beteiligt ist, umfassend Anwenden einer Test-Verbindung an der Maus oder Körperteils davon nach Anspruch 9 und Bestimmen der Verbesserung der Krankheit.

11. Verfahren nach Anspruch 10, wobei die Krankheit metabolisches Syndrom oder eines oder mehrerer Symptome davon ist.

## Revendications

1. Souris transgénique chez laquelle la capacité de sécrétion d'insuline a été augmentée et/ou la tolérance au glucose a été améliorée, par rapport à une souris correspondante non-transgénique, **caractérisée en ce que** chez cette souris est exprimé un gène de récepteur GPR40 exogène, sous le contrôle d'un promoteur insuline-II, ou partie du corps d'une telle souris.

2. Souris conforme à la revendication 1, chez laquelle le récepteur GPR40 exogène présente une séquence d'acides aminés identique ou sensiblement identique à la séquence d'acides aminés présentée en tant que Séquence N° 2, ou partie du corps d'une telle souris.

3. Procédé de recherche par criblage d'un agoniste du récepteur GPR40 ou d'un antagoniste du récepteur GPR40, qui comporte le fait de tester un composé sur une souris conforme à la revendication 1, ou sur une partie du corps d'une telle souris, et le fait d'établir une activité d'agoniste du récepteur GPR40 ou une activité d'antagoniste du récepteur GPR40.

4. Procédé de recherche par criblage d'un médicament permettant (1) de réguler la sécrétion d'insuline et/ou (2) d'agir sur la tolérance au glucose, qui comporte le fait de tester un composé sur une souris conforme à la revendication 1, ou sur une partie du corps d'une telle souris, et le fait de mesurer (1) la sécrétion d'insuline et/ou (2) la tolérance au glucose.

5. Souris conforme à la revendication 1, chez laquelle le récepteur GPR40 exogène est hétérogène à la souris, et chez laquelle souris l'expression du gène du récepteur GPR40 endogène est déficiente, ou partie du corps d'une telle souris.

6. Souris conforme à la revendication 5, chez laquelle le récepteur GPR40 hétérogène est dérivé d'un humain, ou partie du corps d'une telle souris.

7. Procédé de recherche par criblage conforme à la revendication 3, qui est un procédé de recherche par criblage d'un agoniste de récepteur GPR40 hétérogène ou d'un antagoniste de récepteur GPR40 hétérogène, qui comporte le fait de tester un composé sur une souris conforme à la revendication 5, ou sur une partie du corps d'une telle souris, et le fait d'établir une activité d'agoniste du récepteur GPR40 ou une activité d'antagoniste du récepteur GPR40.

8. Procédé de recherche par criblage conforme à la revendication 4, qui est un procédé de recherche par criblage d'un médicament permettant (1) de réguler la sécrétion d'insuline et/ou (2) d'agir sur la tolérance au glucose chez un mammifère hétérogène, qui comporte le fait de tester un composé sur une souris conforme à la revendication 5, ou sur une partie du corps d'une telle souris, et le fait de mesurer (1) la sécrétion d'insuline et/ou (2) la tolérance au glucose.

9. Souris chez laquelle est exprimé un gène de récepteur GPR40 exogène sous le contrôle d'un promoteur insuline II et chez laquelle il y a une ou plusieurs modification(s) de gène, autres que celle du gène du récepteur GPR40, qui produit ou produisent un état identique ou similaire à une maladie dans laquelle est impliquée la régulation de l'activité du récepteur GPR40, ou partie du corps d'une telle souris, laquelle souris résulte du croisement d'une souris chez laquelle est exprimé un gène de récepteur GPR40 exogène sous le contrôle d'un promoteur insuline II et qui est homozygote et d'une souris modèle de maladie chez laquelle il y a une ou plusieurs modification(s) de gène, autres que celle du gène du récepteur GPR40, qui produit ou produisent un état identique ou similaire à une maladie dans laquelle est impliquée la régulation de l'activité du récepteur GPR40.

10. Procédé de recherche par criblage d'une substance prophylactique et/ou thérapeutique pour une maladie dans laquelle est impliquée la régulation de l'activité du récepteur GPR40, qui comporte le fait de tester un composé sur une souris conforme à la revendication 9, ou sur une partie du corps d'une telle souris, et le fait de déterminer s'il y a une amélioration de la maladie.

11. Procédé conforme à la revendication 10, dans lequel la maladie est le syndrome métabolique ou l'un ou plusieurs de ses symptômes.


### SEQUENCE LISTING

<110> Takeda Pharmaceutical Company Limited
<120> Genetically-engineered Animal and Use Thereof
<130> 091026
<150> JP 2006-022913
   <151> 2006-01-31
<160> 22
<170> PatentIn version 3.2
<210> 1
   <211> 900
   <212> DNA
   <213> Homo sapiens
<220>
   <221> CDS
   <222> (1)..(900)
<400> 1
   atg gac ctg ccc ccg cag ctc tcc ttc ggc ctc tat gtg gcc gcc ttt       48
   Met Asp Leu Pro Pro Gln Leu Ser Phe Gly Leu Tyr Val Ala Ala Phe
   1               5                   10                  15
   gcg ctg ggc ttc ccg ctc aac gtc ctg gcc atc cga ggc gcg acg gcc       96
   Ala Leu Gly Phe Pro Leu Asn Val Leu Ala Ile Arg Gly Ala Thr Ala
               20                  25                  30
   cac gcc cgg ctc cgt ctc acc cct agc ctg gtc tac gcc ctg aac ctg      144
   His Ala Arg Leu Arg Leu Thr Pro Ser Leu Val Tyr Ala Leu Asn Leu
           35                  40                  45
   ggc tgc tcc gac ctg ctg ctg aca gtc tct ctg ccc ctg aag gcg gtg      192
   Gly Cys Ser Asp Leu Leu Leu Thr Val Ser Leu Pro Leu Lys Ala Val
       50                  55                  60
   gag gcg cta gcc tcc ggg gcc tgg cct ctg ccg gcc tcg ctg tgc ccc      240
   Glu Ala Leu Ala Ser Gly Ala Trp Pro Leu Pro Ala Ser Leu Cys Pro
   65                  70                  75                  80
   gtc ttc gcg gtg gcc cac ttc ttc cca ctc tat gcc ggc ggg ggc ttc      288
   Val Phe Ala Val Ala His Phe Phe Pro Leu Tyr Ala Gly Gly Gly Phe
                   85                  90                  95
   ctg gcc gcc ctg agt gca ggc cgc tac ctg gga gca gcc ttc ccc ttg      336
   Leu Ala Ala Leu Ser Ala Gly Arg Tyr Leu Gly Ala Ala Phe Pro Leu
               100                 105                 110
   ggc tac caa gcc ttc cgg agg ccg tgc tat tcc tgg ggg gtg tgc gcg      384
   Gly Tyr Gln Ala Phe Arg Arg Pro Cys Tyr Ser Trp Gly Val Cys Ala
           115                 120                 125
   gcc atc tgg gcc ctc gtc ctg tgt cac ctg ggt ctg gtc ttt ggg ttg      432
   Ala Ile Trp Ala Leu Val Leu Cys His Leu Gly Leu Val Phe Gly Leu
       130                 135                 140
   gag gct cca gga ggc tgg ctg gac cac agc aac acc tcc ctg ggc atc       480
   Glu Ala Pro Gly Gly Trp Leu Asp His Ser Asn Thr Ser Leu Gly Ile
   145                 150                 155                 160
   aac aca ccg gtc aac ggc tct ccg gtc tgc ctg gag gcc tgg gac ccg       528
   Asn Thr Pro Val Asn Gly Ser Pro Val Cys Leu Glu Ala Trp Asp Pro
                   165                 170                 175
   gcc tct gcc ggc ccg gcc cgc ttc agc ctc tct ctc ctg ctc ttt ttt       576
   Ala Ser Ala Gly Pro Ala Arg Phe Ser Leu Ser Leu Leu Leu Phe Phe
               180                 185                 190
   ctg ccc ttg gcc atc aca gcc ttc tgc tac gtg ggc tgc ctc cgg gca       624
   Leu Pro Leu Ala Ile Thr Ala Phe Cys Tyr Val Gly Cys Leu Arg Ala
           195                 200                 205
   ctg gcc cgc tcc ggc ctg acg cac agg cgg aag ctg cgg gcc gcc tgg       672
   Leu Ala Arg Ser Gly Leu Thr His Arg Arg Lys Leu Arg Ala Ala Trp
       210                 215                 220
   gtg gcc ggc ggg gcc ctc ctc acg ctg ctg ctc tgc gta gga ccc tac       720
   Val Ala Gly Gly Ala Leu Leu Thr Leu Leu Leu Cys Val Gly Pro Tyr
   225                 230                 235                 240
   aac gcc tcc aac gtg gcc agc ttc ctg tac ccc aat cta gga ggc tcc       768
   Asn Ala Ser Asn Val Ala Ser Phe Leu Tyr Pro Asn Leu Gly Gly Ser
                   245                 250                 255
   tgg cgg aag ctg ggg ctc atc acg ggt gcc tgg agt gtg gtg ctt aat       816
   Trp Arg Lys Leu Gly Leu Ile Thr Gly Ala Trp Ser Val Val Leu Asn
               260                 265                 270
   ccg ctg gtg acc ggt tac ttg gga agg ggt cct ggc ctg aag aca gtg       864
   Pro Leu Val Thr Gly Tyr Leu Gly Arg Gly Pro Gly Leu Lys Thr Val
           275                 280                 285
   tgt gcg gca aga acg caa ggg ggc aag tcc cag aag                       900
   Cys Ala Ala Arg Thr Gln Gly Gly Lys Ser Gln Lys
       290                 295                 300
<210> 2
   <211> 300
   <212> PRT
   <213> Homo sapiens
<400> 2
   Met Asp Leu Pro Pro Gln Leu Ser Phe Gly Leu Tyr Val Ala Ala Phe
   1               5                   10                  15
   Ala Leu Gly Phe Pro Leu Asn Val Leu Ala Ile Arg Gly Ala Thr Ala
               20                  25                  30
   His Ala Arg Leu Arg Leu Thr Pro Ser Leu Val Tyr Ala Leu Asn Leu
           35                  40                  45
   Gly Cys Ser Asp Leu Leu Leu Thr Val Ser Leu Pro Leu Lys Ala Val
       50                  55                  60
   Glu Ala Leu Ala Ser Gly Ala Trp Pro Leu Pro Ala Ser Leu Cys Pro
   65                  70                  75                  80
   Val Phe Ala Val Ala His Phe Phe Pro Leu Tyr Ala Gly Gly Gly Phe
                   85                  90                  95
   Leu Ala Ala Leu Ser Ala Gly Arg Tyr Leu Gly Ala Ala Phe Pro Leu
               100                 105                 110
   Gly Tyr Gln Ala Phe Arg Arg Pro Cys Tyr Ser Trp Gly Val Cys Ala
           115                 120                 125
   Ala Ile Trp Ala Leu Val Leu Cys His Leu Gly Leu Val Phe Gly Leu
       130                 135                 140
   Glu Ala Pro Gly Gly Trp Leu Asp His Ser Asn Thr Ser Leu Gly Ile
   145                 150                 155                 160
   Asn Thr Pro Val Asn Gly Ser Pro Val Cys Leu Glu Ala Trp Asp Pro
                   165                 170                 175
   Ala Ser Ala Gly Pro Ala Arg Phe Ser Leu Ser Leu Leu Leu Phe Phe
               180                 185                 190
   Leu Pro Leu Ala Ile Thr Ala Phe Cys Tyr Val Gly Cys Leu Arg Ala
           195                 200                 205
   Leu Ala Arg Ser Gly Leu Thr His Arg Arg Lys Leu Arg Ala Ala Trp
       210                 215                 220
   Val Ala Gly Gly Ala Leu Leu Thr Leu Leu Leu Cys Val Gly Pro Tyr
   225                 230                 235                 240
   Asn Ala Ser Asn Val Ala Ser Phe Leu Tyr Pro Asn Leu Gly Gly Ser
                   245                 250                 255
   Trp Arg Lys Leu Gly Leu Ile Thr Gly Ala Trp Ser Val Val Leu Asn
               260                 265                 270
   Pro Leu Val Thr Gly Tyr Leu Gly Arg Gly Pro Gly Leu Lys Thr Val
           275                 280                 285
   Cys Ala Ala Arg Thr Gln Gly Gly Lys Ser Gln Lys
   290 295 300
<210> 3
   <211> 48
   <212> DNA
   <213> Artificial
<220>
   <223> Primer
<400> 3
   attagaaagc ttacgcgtga gagatagagg aggagggacc attaagtg 48
<210> 4
   <211> 45
   <212> DNA
   <213> Artificial
<220>
   <223> Primer
<400> 4
   gtcgacacaa taacctggaa gataggctgg gttgaggata gcaaa 45
<210> 5
   <211> 56
   <212> DNA
   <213> Artificial
<220>
   <223> Primer
<400> 5
   attattgtcg accaccatgg acctgccccc gcagctctcc ttcggcctct atgtgg 56
<210> 6
   <211> 24
   <212> DNA
   <213> Artificial
<220>
   <223> Primer
<400> 6
   tatgcacgca aacacaaact ctat 24
<210> 7
   <211> 25
   <212> DNA
   <213> Artificial
<220>
   <223> Primer
<400> 7
   ggagtgtggt gcttaatccg ctggt 25
<210> 8
   <211> 29
   <212> DNA
   <213> Artificial
<220>
   <223> Primer
<400> 8
   agactgcctc ctccttcccg taagtacaa 29
<210> 9
   <211> 18
   <212> DNA
   <213> Artificial
<220>
   <223> Primer
<400> 9
   gcccgcttca gcctctct 18
<210> 10
   <211> 18
   <212> DNA
   <213> Artificial
<220>
   <223> Primer
<400> 10
   gaggcagccc acgtagca 18
<210> 11
   <211> 23
   <212> DNA
   <213> Artificial
<220>
   <223> Probe
<400> 11
   tctgcccttg gccatcacag cct 23
<210> 12
   <211> 22
   <212> DNA
   <213> Artificial
<220>
   <223> Primer
<400> 12
   cagccagtcc cttcccgctt ca 22
<210> 13
   <211> 27
   <212> DNA
   <213> Artificial
<220>
   <223> Primer
<400> 13
   gcaggtccga aatggtcagg tttagca 27
<210> 14
   <211> 19
   <212> DNA
   <213> Artificial
<220>
   <223> Primer
<400> 14
   gcccgccctg cccgtctca 19
<210> 15
   <211> 24
   <212> DNA
   <213> Artificial
<220>
   <223> Primer
<400> 15
   aacgttcgat gctcaccgcc gtca 24
<210> 16
   <211> 17
   <212> DNA
   <213> Artificial
<220>
   <223> Primer
<400> 16
   tttgcgctgg gctttcc 17
<210> 17
   <211> 20
   <212> DNA
   <213> Artificial
<220>
   <223> Primer
<400> 17
   gctgggagtg agtcgcagtt 20
<210> 18
   <211> 21
   <212> DNA
   <213> Artificial
<220>
   <223> Probe
<400> 18
   ccatccgagg cgcagtgtcc c 21
<210> 19
   <211> 22
   <212> DNA
   <213> Artificial
<220>
   <223> Primer
<400> 19
   cgtgaaaaga tgacccagat ca 22
<210> 20
   <211> 20
   <212> DNA
   <213> Artificial
<220>
   <223> Primer
<400> 20
   cacagcctgg atggctacgt 20
<210> 21
   <211> 25
   <212> DNA
   <213> Artificial
<220>
   <223> Probe
<400> 21
   tgagaccttc aacaccccag ccatg 25
<210> 22
   <211> 82
   <212> DNA
   <213> Artificial
<220>
   <223> Synthetic DNA fragment of mouse actin gene
<400> 22
   ccaaccgtga aaagatgacc cagatcatgt ttgagacctt caacacccca gccatgtacg     60
   tagccatcca ggctgtgctg tc                                               82